# EUROPÄISCHE PATENTSCHRIFT

(11) **EP 4 277 511 B1**
(45) Veröffentlichungstag und Bekanntmachung des Hinweises auf die Patenterteilung: **29.07.2026**
(21) Anmeldenummer: 21811055.9
(22) Anmeldetag: 17.11.2021
(51) Int. Cl.: A61B 1/06, A61B 1/07, G02B 23/24

(54) **BELEUCHTUNGSVORRICHTUNG FÜR EIN ENDOSKOPIESYSTEM UND ENDOSKOPIESYSTEM MIT EINER BELEUCHTUNGSVORRICHTUNG**
LIGHTING DEVICE FOR AN ENDOSCOPY SYSTEM, AND ENDOSCOPY SYSTEM HAVING A LIGHTING DEVICE
DISPOSITIF D'ÉCLAIRAGE POUR UN SYSTÈME D'ENDOSCOPIE ET SYSTÈME D'ENDOSCOPIE MUNI D'UN DISPOSITIF D'ÉCLAIRAGE

(30) Priorität: 13.01.2021 DE 102021100572
(43) Veröffentlichungstag der Anmeldung: 22.11.2023
(73) Patentinhaber: HAPPERSBERGER OTOPRONT GmbH, 65329 Hohenstein (DE)
(72) Erfinder: DITTMER, Roland Georg, 55127 Mainz (DE); HAPPERSBERGER, Carlo Rainer, 66329 Hohenstein (DE)
(74) Vertreter: Richly & Ritschel Patentanwälte PartG mbB
(86) Internationale Anmeldenummer: PCT/EP2021/082021
(87) Internationale Veröffentlichungsnummer: WO 2022/152432

(56) Entgegenhaltungen:
- WO-A1-2020/103698
- US-A1- 2009 219 384
- US-A1- 2019 076 008
- US-A1- 2019 110 672
- US-A1- 2020 154 989
- US-B2- 10 610 091

## Beschreibung

Die vorliegende Erfindung betrifft eine Beleuchtungsvorrichtung für ein Endoskopiesystem. Ferner betrifft die vorliegende Erfindung ein Endoskopiesystem mit einer Beleuchtungsvorrichtung.

Endoskopiesysteme ermöglichen visuelle Einblicke in Hohlräume, die lediglich über eine kleine Öffnung zugänglich sind. So ist beispielsweise mittels eines Endoskopiesystems eine visuelle Begutachtung des Körperinneren beispielsweise eines Menschen und auch eines Tieres möglich, wobei die Bildgebung beispielsweise über ein Okular und/oder über eine Digital-Kamera erfolgen kann. Herkömmliche Endoskopiesysteme verwenden als Beleuchtungsvorrichtung zur Beleuchtung des zu untersuchenden Körperbereichs in der Regel sogenanntes Weißlicht, das üblicherweise in eine Lichtleitfaser bzw. ein Faserbündel eingekoppelt wird und über die Lichtleitfaser an den Beobachtungspunkt geleitet wird. Als Weißlicht bezeichnet man Licht, das sowohl rote, grüne und blaue Farbanteile aufweist. Eine breitbandige Weißlichtquelle kann auch sämtliche vom Menschen sichtbare Farbanteile enthalten.

Bei einer Beleuchtung des zu untersuchenden Körperbereichs (beispielsweise eine Mundhöhle oder ein Rachenraum eines Menschen) mittels Weißlicht ist eine natürlich wirkende Bildgebung möglich. Jedoch ist bei einer Beleuchtung mit Weißlicht der Bildkontrast gering, so dass beispielsweise tumoröses Gewebe nur schwierig von gesundem Gewebe unterschieden werden kann.

Um dieses Problem zu lösen, ist es bekannt, anstelle oder zusätzlich von Weißlicht-Bildgebung eine sogenannte Schmalband-Bildgebung anzuwenden, die im Folgenden als NBI (aus dem Englischen: Narrow Band Imaging) abgekürzt wird. Bei der NBI wird lediglich blaues und grünes Licht zur Beleuchtung des zu untersuchenden Gewebes verwendet. Blaues und grünes Licht wird von Hämoglobin besonders gut absorbiert, da Hämoglobin in Wellenlängenbereichen dieser Farbbereiche jeweils Absorptionsmaxima aufweist. Das blaue Licht dringt nicht so tief in das zu untersuchende Gewebe ein, so dass das blaue Licht beispielsweise von Kapillargefäßen in der Schleimhaut stark absorbiert wird, wohingegen das grüne Licht tiefer in das zu untersuchende Gewebe und somit in den submukösen Bereich eindringen kann, wo es teilweise zurückgestreut und von den Blutgefäßen stärker absorbiert wird.

Mit diesem Wirkprinzip entsteht mittels NBI ein wesentlich höherer Kontrast zwischen Blutgefäßen und dem umgebenden Gewebe als bei der Weißlicht-Bildgebung, so dass NBI-Bilder wesentlich kontrastreicher als Weißlichtbilder sind.

NBI bietet bei der frühzeitigen Diagnostik von Kehlkopfkrebs in prä-, intra- und postoperativen Stadien im Vergleich zur Weißlicht-Bildgebung eine verbesserte Effektivität, da tumoröses Gewebe mittels NBI im Verglich zur Weißlicht-Bildgebung in früheren Stadien entdeckt werden kann, so dass Komplikationen und die Zeit, die ein Patient im Krankenhaus verbringt, reduziert werden können. Somit können beispielsweise Hals-Nasen-Ohren-Ärzte mittels NBI Kehlkopfkrebs im Frühstadium noch präziser und zuverlässiger diagnostizieren und behandeln. NBI ist zudem eine sehr genaue Technologie für Nachsorgeuntersuchungen.

Die US 2019 110672 A1 offenbart die Präambel der unabhängigen Ansprüche 1 und 2.

Die EP 2 433 522 B1 beschreibt eine Lichtquellenvorrichtung für ein Endoskopiesystem, die dazu ausgebildet ist, Licht für eine Weißlicht-Bildbebung und Licht für NBI zu emittieren. Hierzu weist die Lichtquellenvorrichtung eine Lichtquelle zum Emittieren von Breitbandlicht im Wellenlängenbereich zwischen 400 nm und 780 nm auf. Zur Ausgabe von schmalbandigem blauen und grünen Licht weist die Lichtquellenvorrichtung drehbare Farbfilter und drehbare Kantenfilter auf, die so gesteuert werden, dass in ersten Zeitschlitzen schmalbandiges grünes Licht und in zweiten Zeitschlitzen schmalbandiges blaues Licht erzeugt wird. Zur Erzeugung eines kontrastreichen Bildes wird ein CCD-Chip mit den Zeitschlitzen des grünen und blauen Lichts synchronisiert.

Dieses aus dem Stand der Technik bekannte System ermöglicht die Kombination von Weißlicht-Bildgebung und NBI. Hierzu ist die Lichtquellenvorrichtung jedoch sehr komplex und weist viele mitunter bewegliche Einzelteile auf, wodurch das System große Abmessungen aufweist und fehleranfällig ist.

Der vorliegenden Erfindung liegt die Aufgabe zugrunde, eine Beleuchtungsvorrichtung für ein Endoskopiesystem bereitzustellen, die einen kompakteren und gleichzeitig stabileren Aufbau aufweist.

Die der vorliegenden Erfindung wird von einer Beleuchtungsvorrichtung mit den Merkmalen von Anspruch 1 sowie von einer Beleuchtungsvorrichtung mit den Merkmalen von Anspruch 2 gelöst. Vorteilhafte Ausgestaltungen der Beleuchtungsvorrichtung sind in den von Ansprüche 1 oder 2 1 abhängigen Ansprüchen beschrieben.

Im Genaueren wird die der vorliegenden Erfindung zugrundeliegende Aufgabe durch eine Beleuchtungsvorrichtung für ein Endoskopiesystem gelöst, die eine erste Leuchteinheit umfasst, die eine erste Lichtquelle und eine erste Sammeloptik zur Bündelung des von der ersten Lichtquelle emittierten Lichts umfasst, wobei die erste Lichtquelle dazu ausgebildet ist, Licht in einem Zentralwellenlängenbereich zwischen 410 nm und 430 nm und einer Halbwertsbreite von weniger als 20 nm zu emittieren. Ferner umfasst die Beleuchtungsvorrichtung eine zweite Leuchteinheit, die eine zweite Lichtquelle und eine zweite Sammeloptik zur Bündelung des von der zweiten Lichtquelle emittierten Lichts umfasst, wobei die zweite Lichtquelle dazu ausgebildet ist, Licht in einem Zentralwellenlängenbereich zwischen 535 nm und 580 nm und einer Halbwertsbreite von weniger als 120 nm zu emittieren. Weiterhin umfasst die Beleuchtungsvorrichtung eine dritte Leuchteinheit, die eine dritte Lichtquelle und eine dritte Sammeloptik zur Bündelung des von der dritten Lichtquelle emittierten Lichts umfasst, wobei die dritte Lichtquelle dazu ausgebildet ist, Licht in einem Zentralwellenlängenbereich zwischen 620 nm und 650 nm und einer Halbwertsbreite von weniger als 30 nm zu emittieren. Außerdem umfasst die Beleuchtungsvorrichtung eine Einkoppeloptik zur Einkopplung des von sämtlichen Leuchteinheiten erzeugten Lichts in das Endoskopiesystem.

Die erfindungsgemäße Beleuchtungsvorrichtung weist den Vorteil auf, dass diese aufgrund der reduzierten Anzahl von Bauteilen einen kompakten Aufbau aufweist und ferner eine verbesserte Robustheit aufweist. Denn durch die Reduzierung bzw. durch den Verzicht auf bewegliche Bauteile ist die Robustheit und somit die Langzeitstabilität der Beleuchtungsvorrichtung erheblich verbessert.

Die erste Lichtquelle ist vorzugsweise als erste Leuchtdiode (LED; Englisch: light emitting-diode) ausgebildet.

Die zweite Lichtquelle ist vorzugsweise als zweite LED ausgebildet. Weiter vorzugsweise weist die zweite Lichtquelle eine zweite LED und einen Lichtkonverter auf, wobei die zweite LED eine Emissionswellenlänge aufweist, die kürzer als die der zweiten Lichtquelle ist, und wobei der Lichtkonverter dazu ausgelegt ist, die von der zweiten LED emittierten Strahlung in den Wellenlängenbereich zwischen 535 nm und 580 nm zu konvertieren.

Die dritte Lichtquelle ist vorzugsweise als dritte Leuchtdiode ausgebildet.

Die erste Sammeloptik ist vorzugsweise als erste Kollimationsoptik ausgebildet. Folglich ist die erste Lichtquelle vorzugsweise im Brennpunkt der ersten Kollimationsoptik angeordnet.

Die zweite Sammeloptik ist vorzugsweise als zweite Kollimationsoptik ausgebildet. Folglich ist die zweite Lichtquelle vorzugsweise im Brennpunkt der zweiten Kollimationsoptik angeordnet.

Die dritte Sammeloptik ist vorzugsweise als dritte Kollimationsoptik ausgebildet. Folglich ist die dritte Lichtquelle vorzugsweise im Brennpunkt der dritten Kollimationsoptik angeordnet.

Vorzugsweise ist die erste Lichtquelle dazu ausgebildet, Licht einem Wellenlängenbereich zwischen 415 nm und 425 nm und weiter vorzugsweise zwischen 418 nm und 422 nm zu emittieren. Nochmals weiter vorzugsweise ist die erste Lichtquelle dazu ausgebildet, Licht mit einer Zentralwellenlänge von 420 nm zu emittieren.

Vorzugsweise weist die erste Lichtquelle eine Strahlungsleistung von zumindest 800 mW auf. Weiter vorzugsweise weist die erste Lichtquelle eine Strahlungsleistung von zumindest 1000 mW auf. Nochmals weiter vorzugsweise weist die erste Lichtquelle eine Strahlungsleistung von zumindest 1200 mW auf.

Vorzugsweise ist die zweite Lichtquelle dazu ausgebildet, Licht einem Wellenlängenbereich zwischen 540 nm und 575 nm zu emittieren.

Vorzugsweise weist die zweite Lichtquelle eine Strahlungsleistung von zumindest 800 mW auf. Weiter vorzugsweise weist die zweite Lichtquelle eine Strahlungsleistung von zumindest 1000 mW auf. Nochmals weiter vorzugsweise weist die zweite Lichtquelle eine Strahlungsleistung von zumindest 1200 mW auf.

Vorzugsweise ist die dritte Lichtquelle dazu ausgebildet, Licht einem Wellenlängenbereich zwischen 625 nm und 645 nm und weiter vorzugsweise zwischen 630 nm und 640 nm zu emittieren. Nochmals weiter vorzugsweise ist die dritte Lichtquelle dazu ausgebildet, Licht mit einer Zentralwellenlänge von 634 nm zu emittieren.

Vorzugsweise weist die dritte Lichtquelle eine Strahlungsleistung von zumindest 800 mW auf. Weiter vorzugsweise weist die dritte Lichtquelle eine Strahlungsleistung von zumindest 1000 mW auf. Nochmals weiter vorzugsweise weist die dritte Lichtquelle eine Strahlungsleistung von zumindest 1200 mW auf.

Vorzugsweise weist die Beleuchtungsvorrichtung eine Steuerungseinrichtung auf, die dazu ausgebildet ist, jede der Lichtquellen unabhängig von den jeweils anderen Lichtquellen zu steuern. So ist es beispielsweise ermöglicht, dass die Steuerungseinrichtung zu einem ersten Zeitpunkt bzw. in einem ersten Zeitintervall die erste Lichtquelle aktiviert und die zweite Lichtquelle und die dritte Lichtquelle deaktiviert. Zu einem zweiten Zeitpunkt bzw. in einem zweiten Zeitintervall aktiviert die Steuerungseinrichtung die zweite Lichtquelle und deaktiviert die erste Lichtquelle und die dritte Lichtquelle. Somit können Blutgefäße in unterschiedlicher Tiefe optisch inspiziert werden.

So ist es ferner ermöglicht, dass die Steuerungseinrichtung zu einem ersten Zeitpunkt bzw. in einem ersten Zeitintervall die erste Lichtquelle und die zweite Lichtquelle aktiviert und die dritte Lichtquelle deaktiviert. Somit ist eine kontrastreiche Bildgebung von Blutgefäße in unterschiedlicher Tiefe ermöglicht.

Ferner kann die Steuerungseinrichtung dazu ausgebildet sein, die erste Lichtquelle, die zweite Lichtquelle und die dritte Lichtquelle zeitgleich zu aktivieren, so dass dann die Beleuchtungsvorrichtung Weißlicht erzeugt.

Vorzugsweise ist die Beleuchtungsvorrichtung derart ausgebildet, dass die zweite Lichtquelle zumindest eine zweite Leuchtdiode und einen Lichtkonverter aufweist.

Die entsprechend ausgebildete Beleuchtungsvorrichtung weist den Vorteil auf, dass der Wellenlängenbereich zwischen 535 nm und 580 nm, in dem Hämoglobin zwei Absorptionsmaxima aufweist, besonders gleichmäßig und mit der richtigen Breitbandigkeit erzeugt werden kann.

Die zweite Leuchtdiode ist vorzugsweise dazu ausgebildet, Licht in einem Zentralwellenlängenbereich zwischen 445 nm und 455 nm und einer Halbwertsbreite von weniger als 25 nm zu emittieren.

Der Lichtkonverter ist vorzugsweise dazu ausgebildet, Licht in einem Zentralwellenlängenbereich zwischen 540 nm und 575 nm zu emittieren, wenn der Lichtkonverter mit dem von der zweiten Leuchtdiode emittierten Licht bestrahlt wird.

Vorzugsweise weist der Lichtkonverter einen Yttrium-Aluminium-Granat auf, der mit Cerium dotiert ist. Weiter vorzugsweise weist der Lichtkonverter einen YAG:Ce - Kristall auf. Weiter vorzugsweise ist ein Teil des Aluminium-Anteils des YAG durch Gadolinium substituiert, wodurch eine erhöhte Emission im Wellenlängenbereich zwischen 540 nm und 575 nm erzielt wird.

Vorzugsweise ist die Beleuchtungsvorrichtung derart ausgebildet, dass das von der ersten Leuchteinheit und/oder von der zweiten Leuchteinheit und/oder von der dritten Leuchteinheit emittierte Licht unmittelbar in die Einkoppeloptik eingekoppelt wird.

Die entsprechend ausgebildete Beleuchtungsvorrichtung ist besonders kompakt aufgebaut und weist eine nochmals reduzierte Anzahl von Einzelkomponenten auf, wodurch die Robustheit der Beleuchtungsvorrichtung nochmals erhöht ist.

Unter einer unmittelbaren Einkopplung in die Einkoppeloptik ist eine Einkopplung des von den Leuchteinheiten emittierten Lichts, im Genaueren von den jeweiligen Lichtquellen der jeweiligen Leuchteinheiten emittierten Lichts, ohne Reflexion über Spiegel im Strahlengang zwischen den jeweiligen Leuchteinheiten und der Einkoppeloptik zu verstehen.

Folglich sind die jeweiligen optischen Achsen der Leuchteinheiten parallel versetzt zueinander angeordnet. Weiterhin sind die optischen Achsen der Leuchteinheiten parallel zur optischen Achse der Einkoppeloptik ausgerichtet.

Die jeweiligen optischen Achsen der Leuchteinheiten sind identische zu den jeweiligen optischen Achsen der jeweiligen Sammeloptiken. D.h., dass z.B. die optische Achse der ersten Leuchteinheit identisch mit der optischen Achse der ersten Sammeloptik ist.

Vorzugsweise ist die Beleuchtungsvorrichtung derart ausgebildet, dass die Beleuchtungsvorrichtung zumindest zwei dichroitische Spiegel aufweist, die zwischen der Einkoppeloptik und den Leuchteinheiten derart angeordnet sind, dass das von den Leuchteinheiten emittierte Licht mittels der zumindest zwei dichroitischen Spiegel in die Einkoppeloptik eingekoppelt wird.

Die entsprechend ausgebildete Beleuchtungsvorrichtung weist den Vorteil auf, dass der Durchmesser der Einkoppeloptik reduziert werden kann.

Eine Einkopplung des von der ersten Leuchteinheit und der zweiten Leuchteinheit und der dritten Leuchteinheit emittierten Lichts mittels dichroitischer Spiegel in die Einkoppeloptik im Sinne der vorliegenden Erfindung ist so zu verstehen, dass das von den Lichtquellen emittierte Licht entweder durch einen der dichroitischen Spiegel transmittiert oder von einem der dichroitischen Spiegel reflektiert wird.

Beispielsweise und nicht beschränkend ist ein erster dichroitischer Spiegel derart ausgebildet, dass dieser das von der ersten Leuchteinheit emittierte Licht reflektiert und das von der zweiten Leuchteinheit und der dritten Leuchteinheit emittierte Licht transmittiert, und dass ein zweiter dichroitischer Spiegel das von der zweiten Leuchteinheit emittierte Licht reflektiert und das von der dritten Leuchteinheit emittierte Licht transmittiert. Selbstverständlich können auch andere als die eben beschriebene Konfiguration der dichroitischen Spiegel verwendet werden, solange mittels der dichroitischen Spiegel die von sämtlichen Leuchteinheiten emittierte Strahlung mittels der dichroitischen Spiegel in die Einkoppeloptik eingekoppelt wird.

Beispielsweise und nicht beschränkend weist der erste dichroitische Spiegel einen Grenzwellenlänge von 520 nm, eine Transmission von mehr als 90% in einem Wellenlängenbereich von 530 nm bis 700 nm und ein Sperrband mit einer Reklektivität von mehr als 90% im Wellenlängenbereich zwischen 400 nm und 510 nm bezogen den verwendeten Einfallswinkel (z.B. 60° oder 45°) auf.

Weiter beispielsweise und nicht beschränkend weist der zweite dichroitische Spiegel einen Grenzwellenlänge von 590 nm, eine Transmission von mehr als 90% in einem Wellenlängenbereich von 600 nm bis 700 nm und ein Sperrband mit einer Reklektivität von mehr als 90% im Wellenlängenbereich zwischen 500 nm und 580 nm bezogen den verwendeten Einfallswinkel (z.B. 60° oder 45°) auf.

Eine erfindungsgemäße Beleuchtungsvorrichtung ist derart ausgebildet, dass die zumindest zwei dichroitischen Spiegel einen Winkel von 120° einschließen.

Vorzugsweise weist bei einer entsprechenden Winkelanordnung der dichroitischen Spiegel die Beleuchtungsvorrichtung vier Leuchteinheiten auf. Zwei der Leuchteinheiten sind dabei so angeordnet, dass jeder der zwei dichroitischen Spiegel zwischen der Einkoppeloptik und einer dieser zwei Leuchteinheiten angeordnet ist, wobei die dichroitischen Spiegel jeweils so ausgebildet sind, das von diesen Leuchteinheiten emittierte Licht zu transmittieren. Die anderen beiden Leuchteinheiten sind so angeordnet, dass deren optische Achsen mit den Leuchteinheiten, deren Licht von den dichroitischen Spiegeln transmittiert wird, jeweils einen Winkel von 120° einschließen. Ferner schließen die optischen Achsen der anderen beiden Leuchteinheiten einen Winkel von 120° miteinander ein.

Die entsprechend ausgebildete Beleuchtungsvorrichtung weist nochmals reduzierte Abmessungen auf. Denn durch eine entsprechende Winkelanordnung der dichroitischen Spiegel können zwei der Leuchteinheiten, deren emittiertes Licht von dichroitischen Spiegel reflektiert wird, platzsparender angeordnet werden, indem eine optische Achse einer dieser Leuchteinheiten senkrecht zu der Flächennormalen des dichroitischen Spiegels verläuft, dessen Licht nicht von diesem dichroitischen Spiegel reflektiert wird, und eine optische Achse der anderen dieser Leuchteinheiten senkrecht zu der Flächennormalen des dichroitischen Spiegels verläuft, dessen Licht nicht von diesem dichroitischen Spiegel reflektiert wird.

Vorzugsweise ist die Beleuchtungsvorrichtung derart ausgebildet, dass die zumindest zwei dichroitischen Spiegel einen Winkel von weniger als 90° einschließen.

Die entsprechend ausgebildete Beleuchtungsvorrichtung ist im Vergleich zu einer Beleuchtungsvorrichtung, bei der die zumindest zwei dichroitischen Spiegel einen Winkel von 90° einschließen, kompakter in ihren Abmessungen. Somit es vereinfacht möglich, die Beleuchtungsvorrichtung beispielsweise in ein Handstück eines Endoskopiesystems zu integrieren.

Ferner sind bei steileren Einfallswinkeln der von den Leuchteinheiten emittierten Strahlung auf einen dichroitischen Spiegel variiert die Übergangswellenlänge der Filterschicht des dichroitischen Spiegels weniger stark als bei flacheren Einfallswinkeln, so dass unter Berücksichtigung der stets vorhandenen Strahldivergenz der von den Leuchteinheiten emittierten Strahlung ein schärferer Übergang zwischen einem Durchlassband und Reflektionsband erzielt wird. Daher lassen sich bei steileren Einfallswinkeln bessere Durchlass- und Reflektionsparameter technisch einfacher realisieren.

Eine erfindungsgemäße Beleuchtungsvorrichtung ist derart ausgebildet, dass die zumindest zwei dichroitischen Spiegel einen Winkel von 60° einschließen.

Die entsprechend ausgebildete Beleuchtungsvorrichtung weist nochmals reduzierte Abmessungen auf. Denn durch eine entsprechende Winkelanordnung der dichroitischen Spiegel können zwei der Leuchteinheiten, deren emittiertes Licht von zumindest einem der dichroitischen Spiegel reflektiert werden, nochmals platzsparender bezüglich der dichroitischen Spiegel angeordnet werden, indem eine optische Achse einer dieser Leuchteinheiten senkrecht zu dem dichroitischen Spiegel verläuft, dessen Licht nicht von diesem dichroitischen Spiegel reflektiert wird, und eine optische Achse der anderen dieser Leuchteinheiten senkrecht zu dem dichroitischen Spiegel verläuft, dessen Licht nicht von diesem dichroitischen Spiegel reflektiert wird. Somit verlaufen die Strahlen des von einer der Leuchteinheiten parallel zu einem dichroitischen Spiegel, der nicht die Strahlung von dieser Leuchteinheit reflektiert. Somit ist ein kompakter Aufbau der Beleuchtungsvorrichtung ermöglicht.

Weiter vorzugsweise ist die Beleuchtungsvorrichtung derart ausgebildet, dass eine der ersten bis dritten Leuchteinheiten bezüglich der zwei dichroitischen Spiegel derart angeordnet ist, dass die optische Achse dieser Leuchteinheit beide dichroitischen Spiegel durchdringt, und jede der übrigen Leuchteinheiten ist bezüglich des dichroitischen Spiegel, der dazu ausgebildet ist, das von dieser Leuchteinheit emittierte Licht zu reflektieren, so angeordnet, dass die optische Achse dieser Leuchteinheit mit der Flächennormalen dieses dichroitischen Spiegels einen Winkel von 30° einschließt.

Die entsprechend ausgebildete Beleuchtungsvorrichtung weist nochmals reduzierte Abmessungen auf, so dass ein kompakter Aufbau der Beleuchtungsvorrichtung ermöglicht ist.

Das Merkmal, gemäß dem die optische Achse einer der Leuchteinheiten beide dichroitische Spiegel durchdringt, bedeutet, dass das von dieser Sammeloptik gebündelte Licht der dieser Sammeloptik zugeordneten Lichtquelle beide dichroitischen Spiegel durchdringt. Folglich sind diese beide dichroitischen Spiegel transparent für die Lichtquelle, die der Sammeloptik zugeordnet ist.

Das Merkmal, gemäß dem jede der übrigen Leuchteinheiten bezüglich des dichroitischen Spiegel, der dazu ausgebildet ist, das von dieser Leuchteinheit emittierte Licht zu reflektieren, so angeordnet ist, dass die optische Achse dieser Leuchteinheit mit der Flächennormalen dieses dichroitischen Spiegels einen Winkel von 30° einschließt, hat bei einer Beleuchtungsvorrichtung mit drei Leuchteinheiten zur Konsequenz, dass
- eine der übrigen Leuchteinheiten bezüglich des dichroitischen Spiegel, der dazu ausgebildet ist, das von dieser Leuchteinheit emittierte Licht zu reflektieren, so angeordnet ist, dass die optische Achse dieser Leuchteinheit mit der Flächennormalen dieses dichroitischen Spiegels einen Winkel von 30° einschließt, und dass
- eine andere der übrigen Leuchteinheiten bezüglich des dichroitischen Spiegel, der dazu ausgebildet ist, das von dieser Leuchteinheit emittierte Licht zu reflektieren, so angeordnet ist, dass die optische Achse dieser Leuchteinheit mit der Flächennormalen dieses dichroitischen Spiegels einen Winkel von 30° einschließt.

Beispielsweise und nicht beschränkend ist die dritte Leuchteinheit bezüglich der zwei dichroitischen Spiegel derart angeordnet, dass die optische Achse der dritten Leuchteinheit beide dichroitischen Spiegel durchdringt und vorzugsweise mit den jeweiligen Flächennormalen der dichroitischen Spiegel jeweils einen Winkel von 30° einschließen.

Beispielsweise und nicht beschränkend ist erste Leuchteinheit bezüglich eines ersten dichroitischen Spiegel, der dazu ausgebildet ist, das von der ersten Leuchteinheit emittierte Licht zu reflektieren, so angeordnet, dass die optische Achse der ersten Leuchteinheit mit der Flächennormalen des ersten dichroitischen Spiegels einen Winkel von 30° einschließt.

Beispielsweise und nicht beschränkend ist zweite Leuchteinheit bezüglich eines zweiten dichroitischen Spiegel, der dazu ausgebildet ist, das von der zweiten Leuchteinheit emittierte Licht zu reflektieren, so angeordnet, dass die optische Achse der zweiten Leuchteinheit mit der Flächennormalen des zweiten dichroitischen Spiegels einen Winkel von 30° einschließt.

Beispielsweise und nicht beschränkend schließen die optische Achse der ersten Leuchteinheit und die optische Achse der zweiten Leuchteinheit einen Winkel von 120° miteinander ein. Beispielsweise und nicht beschränkend schließen die optische Achse der ersten Leuchteinheit und die optische Achse der dritten Leuchteinheit einen Winkel von 120° miteinander ein. Beispielsweise und nicht beschränkend schließen die optische Achse der dritten Leuchteinheit und die optische Achse der zweiten Leuchteinheit einen Winkel von 120° miteinander ein.

Vorzugsweise ist die Beleuchtungsvorrichtung derart ausgebildet, dass diese
- eine vierte Leuchteinheit aufweist, die eine vierte Lichtquelle und eine vierte Sammeloptik zur Bündelung des von der vierten Lichtquelle emittierten Lichts umfasst, wobei die vierte Lichtquelle dazu ausgebildet ist, Licht in einem Zentralwellenlängenbereich zwischen 470 nm und 490 nm und einer Halbwertsbreite von weniger als 30 nm zu emittieren, und/oder
- eine fünfte Leuchteinheit aufweist, die eine fünfte Lichtquelle und eine fünfte Sammeloptik zur Bündelung des von der fünften Lichtquelle emittierten Lichts umfasst, wobei die fünfte Lichtquelle dazu ausgebildet ist, Licht in einem Zentralwellenlängenbereich zwischen 580 nm und 620 nm und einer Halbwertsbreite von weniger als 120 nm zu emittieren; und/oder
- eine sechste Leuchteinheit aufweist, die eine sechste Lichtquelle und eine sechste Sammeloptik zur Bündelung des von der sechsten Lichtquelle emittierten Lichts umfasst, wobei die sechste Lichtquelle dazu ausgebildet ist, Licht in einem Zentralwellenlängenbereich zwischen 650 nm und 670 nm und einer Halbwertsbreite von weniger als 30 nm zu emittieren.

Durch eine entsprechende Ausbildung der Beleuchtungsvorrichtung ist es ermöglicht, dass bei gleichzeitiger Aktivierung sämtlicher Leuchteinheiten ein gleichmäßigeres Spektrum im sichtbaren Bereich erzeugt wird, so dass eine verbesserte Beleuchtung des zu untersuchenden Gewebes im Weißlichtbeleuchtungsmodus erreicht wird.

Die vierte Lichtquelle ist vorzugsweise als vierte Leuchtdiode ausgebildet.

Die fünfte Lichtquelle ist vorzugsweise als fünfte LED ausgebildet. Weiter vorzugsweise weist die fünfte Lichtquelle eine fünfte LED und einen Lichtkonverter auf, wobei die fünfte LED eine Emissionswellenlänge aufweist, die kürzer als die der fünften Lichtquelle ist, und wobei der Lichtkonverter dazu ausgelegt ist, die von der fünfen LED emittierten Strahlung in eine Strahlung in den Wellenlängenbereich zwischen 580 nm und 620 nm zu konvertieren.

Alternativ weist die Beleuchtungsvorrichtung eine fünfte Leuchteinheit auf, die eine fünfte Lichtquelle und eine fünfte Sammeloptik zur Bündelung des von der fünften Lichtquelle emittierten Lichts umfasst, wobei die fünfte Lichtquelle dazu ausgebildet ist, Licht in einem Zentralwellenlängenbereich zwischen 535 nm und 580 nm und einer Halbwertsbreite von weniger als 120 nm zu emittieren. Bei dieser alternativen Beleuchtungsvorrichtung ist die fünfte Lichtquelle vorzugsweise als fünfte LED ausgebildet. Weiter vorzugsweise weist die fünfte Lichtquelle eine fünfte LED und einen Lichtkonverter auf, wobei die fünfte LED eine Emissionswellenlänge aufweist, die kürzer als die der fünften Lichtquelle ist, und wobei der Lichtkonverter dazu ausgelegt ist, die von der fünfen LED emittierten Strahlung in eine Strahlung in den Wellenlängenbereich zwischen 535 nm und 580 nm zu konvertieren. Der Aufbau dieser alternativen fünften Leuchteinheit entspricht vorzugsweise dem Aufbau der zweiten Leuchteinheit.

Die sechste Lichtquelle ist vorzugsweise als sechste Leuchtdiode ausgebildet.

Die vierte Sammeloptik ist vorzugsweise als vierte Kollimationsoptik ausgebildet. Folglich ist die vierte Lichtquelle vorzugsweise im Brennpunkt der vierten Kollimationsoptik angeordnet.

Die fünfte Sammeloptik ist vorzugsweise als fünfte Kollimationsoptik ausgebildet. Folglich ist die fünfte Lichtquelle vorzugsweise im Brennpunkt der fünften Kollimationsoptik angeordnet.

Die sechste Sammeloptik ist vorzugsweise als sechste Kollimationsoptik ausgebildet. Folglich ist die sechste Lichtquelle vorzugsweise im Brennpunkt der sechsten Kollimationsoptik angeordnet.

Vorzugsweise weist die vierte Lichtquelle eine Strahlungsleistung von zumindest 800 mW auf. Weiter vorzugsweise weist die vierte Lichtquelle eine Strahlungsleistung von zumindest 1000 mW auf. Nochmals weiter vorzugsweise weist die vierte Lichtquelle eine Strahlungsleistung von zumindest 1200 mW auf.

Vorzugsweise ist die fünfte Lichtquelle dazu ausgebildet, Licht einem Wellenlängenbereich zwischen 540 nm und 575 nm zu emittieren.

Vorzugsweise weist die fünfte Lichtquelle eine Strahlungsleistung von zumindest 800 mW auf. Weiter vorzugsweise weist die fünfte Lichtquelle eine Strahlungsleistung von zumindest 1000 mW auf. Nochmals weiter vorzugsweise weist die fünfte Lichtquelle eine Strahlungsleistung von zumindest 1200 mW auf.

Vorzugsweise weist die sechste Lichtquelle eine Strahlungsleistung von zumindest 800 mW auf. Weiter vorzugsweise weist die sechste Lichtquelle eine Strahlungsleistung von zumindest 1000 mW auf. Nochmals weiter vorzugsweise weist die sechste Lichtquelle eine Strahlungsleistung von zumindest 1200 mW auf.

Vorzugsweise weist die Beleuchtungsvorrichtung eine Steuerungseinrichtung auf, die dazu ausgebildet ist, jede der Lichtquellen unabhängig von den jeweils anderen Lichtquellen zu steuern. So ist es beispielsweise ermöglicht, dass die Steuerungseinrichtung zu einem ersten Zeitpunkt bzw. in einem ersten Zeitintervall die erste Lichtquelle und die vierte Lichtquelle aktiviert und die übrigen Lichtquellen deaktiviert. Zu einem zweiten Zeitpunkt bzw. in einem zweiten Zeitintervall aktiviert die Steuerungseinrichtung die zweite Lichtquelle und die fünfte Lichtquelle und deaktiviert die übrigen Lichtquellen. Somit können Blutgefäße in unterschiedlicher Tiefe optisch inspiziert werden.

So ist es ferner ermöglicht, dass die Steuerungseinrichtung zu einem ersten Zeitpunkt bzw. in einem ersten Zeitintervall die erste Lichtquelle, die vierte Lichtquelle, die zweite Lichtquelle und die fünfte Lichtquelle aktiviert und die übrigen Lichtquellen deaktiviert. Somit ist eine kontrastreiche Bildgebung von Blutgefäße in unterschiedlicher Tiefe ermöglicht.

Ferner kann die Steuerungseinrichtung dazu ausgebildet sein, sämtliche Lichtquellen zeitgleich zu aktivieren, so dass dann die Beleuchtungsvorrichtung Weißlicht erzeugt.

Vorzugsweise ist die Beleuchtungsvorrichtung derart ausgebildet, dass die fünfte Lichtquelle zumindest eine Leuchtdiode und einen Lichtkonverter aufweist.

Die fünfte Leuchtdiode ist vorzugsweise dazu ausgebildet, Licht in einem Zentralwellenlängenbereich zwischen 445 nm und 455 nm und einer Halbwertsbreite von weniger als 25 nm zu emittieren.

Der Lichtkonverter ist vorzugsweise dazu ausgebildet, Licht in einem Zentralwellenlängenbereich zwischen 580 nm und 620 nm zu emittieren, wenn der Lichtkonverter mit dem von der fünften Leuchtdiode emittierten Licht bestrahlt wird.

Weiter vorzugsweise ist die Beleuchtungsvorrichtung derart ausgebildet, dass die Beleuchtungsvorrichtung die vierte Leuchteinheit, die fünfte Leuchteinheit und die sechste Leuchteinheit und zumindest zwei weitere dichroitische Spiegel aufweist, die zwischen der Einkoppeloptik und den vierten bis sechsten Leuchteinheiten derart angeordnet sind, dass das von den vierten bis sechsten Leuchteinheiten emittierte Licht mittels der zumindest zwei weiteren dichroitischen Spiegel in die Einkoppeloptik eingekoppelt wird.

Die entsprechend ausgebildete Beleuchtungsvorrichtung weist den Vorteil auf, dass der Durchmesser der Einkoppeloptik trotz der großen Anzahl von Leuchteinheiten reduziert werden kann.

Eine Einkopplung des von der vierten Leuchteinheit und der fünften Leuchteinheit und der sechsten Leuchteinheit emittierten Lichts mittels dichroitischer Spiegel in die Einkoppeloptik im Sinne der vorliegenden Erfindung ist so zu verstehen, dass das von den Lichtquellen emittierte Licht entweder durch einen der dichroitischen Spiegel transmittiert oder von einem der dichroitischen Spiegel reflektiert wird.

Beispielsweise und nicht beschränkend ist ein dritter dichroitischer Spiegel derart ausgebildet, dass dieser das von der vierten Leuchteinheit emittierte Licht reflektiert und das von der fünften Leuchteinheit und der sechsten Leuchteinheit emittierte Licht transmittiert, und dass ein vierter dichroitischer Spiegel das von der fünften Leuchteinheit emittierte Licht reflektiert und das von der sechsten Leuchteinheit emittierte Licht transmittiert. Selbstverständlich können auch andere als die eben beschriebene Konfiguration der dichroitischen Spiegel verwendet werden, solange mittels der dichroitischen Spiegel die von sämtlichen Leuchteinheiten emittierte Strahlung mittels der dichroitischen Spiegel in die Einkoppeloptik eingekoppelt wird.

Beispielsweise und nicht beschränkend weist der dritte dichroitische Spiegel einen Grenzwellenlänge von 520 nm, eine Transmission von mehr als 90% in einem Wellenlängenbereich von 530 nm bis 700 nm und ein Sperrband mit einer Reklektivität von mehr als 90% im Wellenlängenbereich zwischen 400 nm und 510 nm bezogen den verwendeten Einfallswinkel (z.B. 60° oder 45°) auf.

Weiter beispielsweise und nicht beschränkend weist der vierte dichroitische Spiegel einen Grenzwellenlänge von 590 nm, eine Transmission von mehr als 90% in einem Wellenlängenbereich von 600 nm bis 700 nm und ein Sperrband mit einer Reklektivität von mehr als 90% im Wellenlängenbereich zwischen 500 nm und 580 nm bezogen den verwendeten Einfallswinkel (z.B. 60° oder 45°) auf.

Vorzugsweise sind die dritten und vierten dichroitischen Spiegel so zueinander angeordnet, dass diese einen Winkel von 90° einschließen, also senkrecht zueinander angeordnet sind.

Die ersten bis sechsten Leuchteinheiten sind vorzugsweise so angeordnet, dass die optischen Achsen der ersten bis sechsten Leuchteinheiten in einer Ebene angeordnet sind. Folglich sind dann die ersten bis vierten dichroitischen Spiegel derart angeordnet, dass die Strahlung der ersten bis sechsten Leuchteinheiten entsprechend der oberen Beschreibung von den ersten bis vierten dichroitischen Spiegeln entweder reflektiert oder transmittiert wird. Weiter vorzugsweise weist die Beleuchtungsvorrichtung zusätzlich zu den ersten bis sechsten Leuchteinheiten siebte bis zwölfte Leuchteinheiten auf. Die entsprechend ausgebildete Beleuchtungsvorrichtung weist ferner zusätzlich zu den ersten bis vierten dichroitischen Spiegeln fünfte bis achte dichroitische Spiegel auf. Vorzugsweise sind die siebten bis zwölften Leuchteinheiten bezüglich der ersten bis sechsten Leuchteinheiten derart angeordnet, dass die jeweiligen optischen Achsen der siebten bis zwölften Leuchteinheiten parallel versetzt zu den jeweiligen optischen Achsen der ersten bis sechsten Leuchteinheiten verlaufen. D.h., dass die optische Achse der siebten Leuchteinheit parallel versetzt zur optischen Achse der ersten Leuchteinheit, die optische Achse der achten Leuchteinheit parallel versetzt zur optischen Achse der zweiten Leuchteinheit, usw. angeordnet sind. Die fünften bis achten dichroitischen Spiegel sind vorzugsweise derart angeordnet, dass die Strahlung der siebten bis zwölften Leuchteinheiten entsprechend von den fünften bis achten dichroitischen Spiegeln entweder reflektiert oder transmittiert wird, so dass die Strahlung der siebten bis zwölften Leuchteinheiten in die Einkoppeloptik eingekoppelt wird. Die fünften bis achten dichroitischen Spiegel sind vorzugsweise hinsichtlich ihrer Winkelanordnung zueinander entsprechend den Winkelanordnungen der ersten bis vierten dichroitischen Spiegel zueinander angeordnet bzw. ausgerichtet. D.h., dass die Winkelanordnung des fünften dichroitischen Spiegels zum sechsten dichroitischen Spiegel der Winkelanordnung des ersten dichroitischen Spiegels zum zweiten dichroitischen Spiegel entspricht, und dass die Winkelanordnung des siebten dichroitischen Spiegels zum achten dichroitischen Spiegel der Winkelanordnung des dritten dichroitischen Spiegels zum vierten dichroitischen Spiegel entspricht. Die siebten bis zwölften Leuchteinheiten sind vorzugsweise entsprechend den ersten bis sechsten Leuchteinheiten ausgebildet. D.h., dass die siebte Leuchteinheit entsprechend der ersten Leuchteinheit, die achte Leuchteinheit entsprechend der zweiten Leuchteinheit, usw. ausgebildet sind. Es ist jedoch auch möglich, dass die siebten bis zwölften Leuchteinheiten nicht entsprechend der ersten bis sechsten Leuchteinheiten ausgebildet sind.

Eine entsprechend ausgebildete Beleuchtungsvorrichtung weist den Vorteil auf, dass diese trotz kompakter Bauweise eine große Lichtintensität in den jeweiligen Wellenlängenbereichen aufweist. Denn der Durchmesser der Einkoppeloptik wird optimal ausgenutzt, indem die ersten bis sechsten Leuchteinheiten in einer ersten Ebene und die siebten bis zwölften Leuchteinheiten in einer zur ersten Ebene parallel versetzten zweiten Ebene angerordnet sind.

Vorzugsweise ist die Beleuchtungsvorrichtung derart ausgebildet, dass die zumindest zwei weiteren dichroitischen Spiegel einen Winkel von weniger als 90° einschließen.

Die entsprechend ausgebildete Beleuchtungsvorrichtung ist im Vergleich zu einer Beleuchtungsvorrichtung, bei der die zumindest zwei weiteren dichroitischen Spiegel einen Winkel von 90° einschließen, kompakter in ihren Abmessungen. Somit es vereinfacht möglich, die Beleuchtungsvorrichtung beispielsweise in ein Handstück eines Endoskopiesystems zu integrieren.

Ferner sind bei steileren Einfallswinkeln der von den Leuchteinheiten emittierten Strahlung auf einen dichroitischen Spiegel variiert die Übergangswellenlänge der Filterschicht des dichroitischen Spiegels weniger stark als bei flacheren Einfallswinkeln, so dass unter Berücksichtigung der stets vorhandenen Strahldivergenz der von den Leuchteinheiten emittierten Strahlung ein schärferer Übergang zwischen einem Durchlassband und Reflektionsband erzielt wird. Daher lassen sich bei steileren Einfallswinkeln bessere Durchlass- und Reflektionsparameter technisch einfacher realisieren.

Vorzugsweise ist die Beleuchtungsvorrichtung derart ausgebildet, dass die zumindest zwei weiteren dichroitischen Spiegel einen Winkel von 60° einschließen.

Die entsprechend ausgebildete Beleuchtungsvorrichtung weist nochmals reduzierte Abmessungen auf. Denn durch eine entsprechende Winkelanordnung der weiteren dichroitischen Spiegel können zwei der Leuchteinheiten, deren emittiertes Licht von zumindest einem der weiteren dichroitischen Spiegel reflektiert werden, nochmals platzsparender bezüglich der weiteren dichroitischen Spiegel angeordnet werden, indem eine optische Achse einer dieser Leuchteinheiten senkrecht zu dem weiteren dichroitischen Spiegel verläuft, dessen Licht nicht von diesem weiteren dichroitischen Spiegel reflektiert wird, und eine optische Achse der anderen dieser Leuchteinheiten senkrecht zu dem weiteren dichroitischen Spiegel verläuft, dessen Licht nicht von diesem weiteren dichroitischen Spiegel reflektiert wird.

Weiter vorzugsweise ist die Beleuchtungsvorrichtung derart ausgebildet, dass eine der vierten bis sechsten Leuchteinheiten bezüglich der zwei weiteren dichroitischen Spiegel derart angeordnet ist, dass die optische Achse dieser Leuchteinheit beide weiteren dichroitischen Spiegel durchdringt, wobei jede der übrigen vierten bis sechsten Leuchteinheiten bezüglich des weiteren dichroitischen Spiegels, der dazu ausgebildet ist, das von dieser Leuchteinheit emittierte Licht zu reflektieren, so angeordnet ist, dass die optische Achse dieser Leuchteinheit mit der Flächennormalen dieses weiteren dichroitischen Spiegels einen Winkel von 30° einschließt.

Die entsprechend ausgebildete Beleuchtungsvorrichtung weist nochmals reduzierte Abmessungen auf, so dass ein kompakter Aufbau der Beleuchtungsvorrichtung ermöglicht ist.

Das Merkmal, gemäß dem die optische Achse einer der vierten bis sechsten Leuchteinheiten beide weiteren dichroitische Spiegel durchdringt, bedeutet, dass das von dieser Sammeloptik gebündelte Licht der dieser Sammeloptik zugeordneten Lichtquelle beide weiteren dichroitischen Spiegel durchdringt. Folglich sind diese beiden weiteren dichroitischen Spiegel transparent für die Lichtquelle, die der Sammeloptik zugeordnet ist.

Das Merkmal, gemäß dem jede der übrigen vierten bis sechsten Leuchteinheiten bezüglich des weiteren dichroitischen Spiegel, der dazu ausgebildet ist, das von dieser Leuchteinheit emittierte Licht zu reflektieren, so angeordnet ist, dass die optische Achse dieser Leuchteinheit mit der Flächennormalen dieses weiteren dichroitischen Spiegels einen Winkel von 30° einschließt, hat bei einer Beleuchtungsvorrichtung mit drei weiteren (vierten bis sechsten) Leuchteinheiten zur Konsequenz, dass
- eine der übrigen (vierten bis sechsten) Leuchteinheiten bezüglich des weiteren dichroitischen Spiegel, der dazu ausgebildet ist, das von dieser Leuchteinheit emittierte Licht zu reflektieren, so angeordnet ist, dass die optische Achse dieser Leuchteinheit mit der Flächennormalen dieses weiteren dichroitischen Spiegels einen Winkel von 30° einschließt, und dass
- eine andere der übrigen (vierten bis sechsten) Leuchteinheiten bezüglich des weiteren dichroitischen Spiegel, der dazu ausgebildet ist, das von dieser Leuchteinheit emittierte Licht zu reflektieren, so angeordnet ist, dass die optische Achse dieser Leuchteinheit mit der Flächennormalen dieses weiteren dichroitischen Spiegels einen Winkel von 30° einschließt.

Beispielsweise und nicht beschränkend ist die dritte Leuchteinheit bezüglich der zwei dichroitischen Spiegel derart angeordnet, dass die optische Achse der dritten Leuchteinheit beide dichroitischen Spiegel durchdringt und vorzugsweise mit den jeweiligen Flächennormalen der dichroitischen Spiegel jeweils einen Winkel von 30° einschließen.

Beispielsweise und nicht beschränkend ist vierte Leuchteinheit bezüglich eines dritten dichroitischen Spiegel, der dazu ausgebildet ist, das von der vierten Leuchteinheit emittierte Licht zu reflektieren, so angeordnet, dass die optische Achse der vierten Leuchteinheit mit der Flächennormalen des dritten dichroitischen Spiegels einen Winkel von 30° einschließt.

Beispielsweise und nicht beschränkend ist fünfte Leuchteinheit bezüglich eines vierten dichroitischen Spiegel, der dazu ausgebildet ist, das von der fünften Leuchteinheit emittierte Licht zu reflektieren, so angeordnet, dass die optische Achse der fünften Leuchteinheit mit der Flächennormalen des vierten dichroitischen Spiegels einen Winkel von 30° einschließt.

Beispielsweise und nicht beschränkend schließen die optische Achse der vierten Leuchteinheit und die optische Achse der fünften Leuchteinheit einen Winkel von 120° miteinander ein. Beispielsweise und nicht beschränkend schließen die optische Achse der vierten Leuchteinheit und die optische Achse der sechsten Leuchteinheit einen Winkel von 120° miteinander ein. Beispielsweise und nicht beschränkend schließen die optische Achse der sechsten Leuchteinheit und die optische Achse der fünften Leuchteinheit einen Winkel von 120° miteinander ein.

Vorzugsweise ist die Beleuchtungsvorrichtung derart ausgebildet, dass die Beleuchtungsvorrichtung eine Weißlicht-Leuchteinheit aufweist, die eine Weißlichtquelle und eine Weißlicht-Sammeloptik zur Bündelung des von der Weißlichtquelle emittierten Lichts umfasst, wobei die Weißlichtquelle dazu ausgebildet ist, breitbandiges Weißlicht zu emittieren.

Die entsprechend ausgebildete Beleuchtungsvorrichtung weist den Vorteil auf, dass Weißlicht mit einer größeren Intensität erzeugbar ist, so dass die entsprechende Weißlicht-Bildgebung verbessert ermöglicht ist.

Die Weißlichtquelle ist vorzugsweise als Weißlicht-Leuchtdiode ausgebildet.

Die erste Weißlicht-Sammeloptik ist vorzugsweise als Weißlicht-Kollimationsoptik ausgebildet. Folglich ist die Weißlichtquelle vorzugsweise im Brennpunkt der Weißlicht-Kollimationsoptik angeordnet.

Vorzugsweise ist die Weißlichtquelle dazu ausgebildet, Licht bei einem primären Emissionsmaximum bei 460 nm und bei einem sekundären Emissionsmaximum bei 580 nm zu emittieren.

Vorzugsweise weist die Weißlichtquelle eine Strahlungsleistung von zumindest 800 mW auf. Weiter vorzugsweise weist die Weißlichtquelle eine Strahlungsleistung von zumindest 1000 mW auf. Nochmals weiter vorzugsweise weist die Weißlichtquelle eine Strahlungsleistung von zumindest 1200 mW auf. Nochmals weiter vorzugsweise weist die Weißlichtquelle eine Strahlungsleistung von zumindest 2000 mW auf.

Vorzugsweise weist die Beleuchtungsvorrichtung eine Steuerungseinrichtung auf, die dazu ausgebildet ist, bei einer Weißlicht-Bildgebung die Weißlichtquelle und/oder jede der anderen Lichtquellen zu aktivieren.

Weiter vorzugsweise ist die Beleuchtungsvorrichtung derart ausgebildet, dass das von der Weißlicht-Leuchteinheit emittierte Licht unmittelbar in die Einkoppeloptik eingekoppelt wird.

Die entsprechend ausgebildete Beleuchtungsvorrichtung ist besonders kompakt aufgebaut und weist eine nochmals reduzierte Anzahl von Einzelkomponenten auf, wodurch die Robustheit der Beleuchtungsvorrichtung nochmals erhöht ist.

Unter einer unmittelbaren Einkopplung in die Einkoppeloptik ist eine Einkopplung des von der Weißlicht-Leuchteinheit emittierten Lichts, im Genaueren von der Weißlichtquelle emittierten Lichts, ohne Reflexion über Spiegel im Strahlengang zwischen der Weißlicht-Leuchteinheit und der Einkoppeloptik zu verstehen.

Folglich ist die optische Achse der Weißlicht-Leuchteinheit parallel zur optischen Achse der Einkoppeloptik ausgerichtet.

Weiter vorzugsweise ist die Beleuchtungsvorrichtung derart ausgebildet, dass die Beleuchtungsvorrichtung zumindest zwei dichroitische Spiegel aufweist, wobei einer der dichroitischen Spiegel zwischen der Einkoppeloptik und den Leuchteinheiten und der andere dichroitische Spiegel zwischen der Weißlicht-Leuchteinheit und den Leuchteinheiten und der Einkoppeloptik derart angeordnet sind, dass das von den Leuchteinheiten und von der Weißlicht-Leuchteinheit emittierte Licht mittels der zumindest zwei dichroitischen Spiegel in die Einkoppeloptik eingekoppelt wird.

Die entsprechend ausgebildete Beleuchtungsvorrichtung weist den Vorteil auf, dass der Durchmesser der Einkoppeloptik reduziert werden kann.

Eine Einkopplung des von den ersten bis dritten Leuchteinheiten und der Weißlicht-Leuchteinheit emittierten Lichts mittels dichroitischer Spiegel in die Einkoppeloptik im Sinne der vorliegenden Erfindung ist so zu verstehen, dass das von den Lichtquellen emittierte Licht entweder durch einen der dichroitischen Spiegel transmittiert oder von einem der dichroitischen Spiegel reflektiert wird.

Beispielsweise und nicht beschränkend ist ein erster dichroitischer Spiegel derart ausgebildet, dass dieser das von der ersten Leuchteinheit emittierte Licht reflektiert und das von der Weißlicht-Leuchteinheit emittierte Licht transmittiert, und dass ein zweiter dichroitischer Spiegel das von der zweiten Leuchteinheit emittierte Licht reflektiert und das von der dritten Leuchteinheit emittierte Licht transmittiert. Selbstverständlich können auch andere als die eben beschriebene Konfiguration der dichroitischen Spiegel verwendet werden, solange mittels der dichroitischen Spiegel die von sämtlichen Leuchteinheiten emittierte Strahlung mittels der dichroitischen Spiegel in die Einkoppeloptik eingekoppelt wird.

Die ersten bis dritten Leuchteinheiten und die Weißlicht-Leuchteinheit sind vorzugsweise so angeordnet, dass die optischen Achsen der ersten bis dritten Leuchteinheiten und der Weißlicht-Leuchteinheit in einer Ebene angeordnet sind. Folglich sind dann die zwei dichroitischen Spiegel derart angeordnet, dass die Strahlung der ersten bis dritten Leuchteinheiten und der Weilicht-Leuchteinheit entsprechend der oberen Beschreibung von den zwei dichroitischen Spiegeln entweder reflektiert oder transmittiert wird. Weiter vorzugsweise weist die Beleuchtungsvorrichtung zusätzlich zu den ersten bis dritten Leuchteinheiten vierte bis sechste Leuchteinheiten auf und zusätzlich zur ersten Weißlicht-Leuchteinheit eine zweite Weißlicht-Leuchteinheit auf. Die entsprechend ausgebildete Beleuchtungsvorrichtung weist ferner zusätzlich zu den zwei dichroitischen Spiegeln, die als erste und zweite dichroitische Spiegel bezeichnet werden können, zwei weitere dichroitische Spiegel, nämlich einen dritten und einen vierten dichroitischen Spiegel auf. Vorzugsweise sind die vierten bis sechsten Leuchteinheiten bezüglich der ersten bis dritten Leuchteinheiten derart angeordnet, dass die jeweiligen optischen Achsen der vierten bis sechsten Leuchteinheiten parallel versetzt zu den jeweiligen optischen Achsen der ersten bis dritten Leuchteinheiten verlaufen. Weiter vorzugsweise ist die zweite Weißlicht-Leuchteinheit bezüglich der ersten Weißlicht-Leuchteinheit derart angeordnet, dass die optische Achse der zweiten Weißlicht-Leuchteinheit parallel versetzt zu der optischen Achse der ersten Weißlicht-Leuchteinheit verläuft. D.h., dass die optische Achse der vierten Leuchteinheit parallel versetzt zur optischen Achse der ersten Leuchteinheit, die optische Achse der fünften Leuchteinheit parallel versetzt zur optischen Achse der zweiten Leuchteinheit, die optische Achse der sechsten Leuchteinheit parallel versetzt zur optischen Achse der dritten Leuchteinheit, und die optische Achse der zweiten Weißlicht-Leuchteinheit parallel versetzt zur optischen Achse der ersten Weißlicht-Leuchteinheit angeordnet sind. Der dritte und vierte dichroitische Spiegel sind vorzugsweise derart angeordnet, dass die Strahlung der vierten bis sechsten Leuchteinheiten und der zweiten Weißlicht-Leuchteinheit entsprechend von dem dritten oder vierten dichroitischen Spiegel entweder reflektiert oder transmittiert wird, so dass die Strahlung der vierten bis sechsten Leuchteinheiten und der zweiten Weißlicht-Leuchteinheit in die Einkoppeloptik eingekoppelt wird. Der dritte und vierte dichroitische Spiegel sind vorzugsweise hinsichtlich ihrer Winkelanordnung zueinander entsprechend der Winkelanordnung des ersten und zweiten dichroitischen Spiegels zueinander angeordnet bzw. ausgerichtet. D.h., dass die Winkelanordnung des dritten dichroitischen Spiegels zum vierten dichroitischen Spiegel der Winkelanordnung des ersten dichroitischen Spiegels zum zweiten dichroitischen Spiegel entspricht. Die vierten bis sechsten Leuchteinheiten sind vorzugsweise entsprechend den ersten bis dritten Leuchteinheiten ausgebildet. D.h., dass die vierte Leuchteinheit entsprechend der ersten Leuchteinheit, die fünfte Leuchteinheit entsprechend der zweiten Leuchteinheit, die sechste Leuchteinheit entsprechend der dritten Leuchteinheit ausgebildet sind. Es ist jedoch auch möglich, dass die vierten bis sechsten Leuchteinheiten nicht entsprechend der ersten bis dritten Leuchteinheiten ausgebildet sind. Weiter vorzugsweise ist die zweite Weißlicht-Leuchteinheit entsprechend der ersten Weißlicht-Leuchteinheit ausgebildet.

Eine entsprechend ausgebildete Beleuchtungsvorrichtung weist den Vorteil auf, dass diese trotz kompakter Bauweise eine große Lichtintensität in den jeweiligen Wellenlängenbereichen und im Weißlichtbereich aufweist. Denn der Durchmesser der Einkoppeloptik wird optimal ausgenutzt, indem die ersten bis dritten Leuchteinheiten und die erste Weißlicht-Leuchteinheit in einer ersten Ebene und die vierten bis sechsten Leuchteinheiten und die zweite Weißlicht-Leuchteinheit in einer zur ersten Ebene parallel versetzten zweiten Ebene angerordnet sind.

Weiter vorzugsweise ist die Beleuchtungsvorrichtung derart ausgebildet, dass eine optische Achse der Weißlicht-Leuchteinheit und eine optische Achse einer der ersten bis dritten Leuchteinheiten parallel zueinander verlaufen.

Die der vorliegenden Erfindung zugrundeliegende Aufgabe wird ferner durch ein Endoskopiesystem mit den Merkmalen von Anspruch 19 gelöst. Vorteilhafte Ausgestaltungen des Endoskopiesystems sind in den von Anspruch 19 abhängigen Ansprüchen beschrieben.

Im Genaueren wird die der vorliegenden Erfindung zugrundeliegende Aufgabe auch durch ein Endoskopiesystem gelöst, das eine Beleuchtungsvorrichtung nach einer der oben beschriebenen Ausführungsformen und eine Steuerungseinrichtung umfasst, die dazu ausgebildet ist, jede der Lichtquellen unabhängig von den jeweils anderen Lichtquellen zu steuern.

Das erfindungsgemäße Endoskopiesystem weist den Vorteil auf, dass dieses aufgrund der reduzierten Anzahl von Bauteilen in der Beleuchtungsvorrichtung einen kompakten Aufbau aufweist und ferner eine verbesserte Robustheit aufweist. Denn durch die Reduzierung bzw. durch den Verzicht auf bewegliche Bauteile in der Beleuchtungsvorrichtung ist die Robustheit und somit die Langzeitstabilität der Beleuchtungsvorrichtung und somit des Endoskopiesystems erheblich verbessert.

Vorzugsweise ist die Beleuchtungsvorrichtung derart ausgebildet, dass die Beleuchtungsvorrichtung in einem Handstück des Endoskopiesystems integriert ist.

Das entsprechend ausgebildete Endoskopiesystem weist unter anderem den Vorteil auf, dass an einem optischen Ausgang des Endoskopiesystems eine erhöhte optische Leistung ankommt, so dass die Bildgebung (sowohl NBI als auch Weißlicht-Bildgebung) verbessert ermöglicht ist. Denn durch die Integration der Beleuchtungsvorrichtung in dem Handstück des Endoskopiesystems treten verminderte Verluste bei der Einkopplung des von der Beleuchtungsvorrichtung erzeugten Lichts auf. Beispielsweise kann eine Einkopplung des von der Beleuchtungsvorrichtung erzeugten Lichts in eine Lichtleitfaser oder in ein Bündel von Lichtleitfasern gänzlich vermieden werden.

Weiter vorzugsweise ist die Beleuchtungsvorrichtung derart ausgebildet, dass eine Kamera im Handstück dazu ausgebildet ist, Bilddaten kabellos zu übertragen.

Das entsprechend ausgebildete Endoskopiesystem ist besonders einfach und komfortabel handhabbar, da auf ein Kabel zwischen dem Handgerät und einer Basisstation des Endoskopiesystems verzichtet werden kann, so dass das Handstück des Endoskopiesystems ohne Einschränkungen durch ein Kabel frei beweglich ist.

Weiter vorzugsweise ist die Beleuchtungsvorrichtung derart ausgebildet, dass in dem Handstück eine elektrische Energiespeichereinrichtung zur Versorgung der Beleuchtungsvorrichtung und/oder der Kamera mit elektrischem Strom integriert ist.

Das entsprechend ausgebildete Endoskopiesystem ist nochmals einfacher und komfortabler handhabbar, da auch auf ein Stromversorgungskabel zwischen dem Handgerät und einer Basisstation des Endoskopiesystems verzichtet werden kann.

Weitere Vorteile, Einzelheiten und Merkmale der Erfindung ergeben sich nachfolgend aus den erläuterten Ausführungsbeispielen. Dabei zeigen im Einzelnen:
- Figur 1:: eine schematische Darstellung einer Beleuchtungsvorrichtung gemäß einer nicht beanspruchten Ausführungsform;
- Figur 2:: eine schematische Darstellung einer Beleuchtungsvorrichtung gemäß einer zweiten Ausführungsform der vorliegenden Erfindung;
- Figur 3:: eine schematische Darstellung einer Beleuchtungsvorrichtung gemäß einer dritten Ausführungsform der vorliegenden Erfindung;
- Figur 4:: eine schematische Darstellung einer Beleuchtungsvorrichtung gemäß einer vierten Ausführungsform der vorliegenden Erfindung;
- Figur 5:: eine schematische Darstellung einer Beleuchtungsvorrichtung gemäß einer fünften Ausführungsform der vorliegenden Erfindung;
- Figur 6A:: eine schematische Darstellung eines erfindungsgemäßen Endoskopiesystems;
- Figur 6B:: das in Figur 6A dargestellte Endoskopiesystem mit weiteren in einer Basisstation angeordneten Funktionseinheiten; und
- Figur 7:: ein Endoskopiesystem gemäß einer weiteren Ausführungsform der vorliegenden Erfindung.

In der nun folgenden Beschreibung bezeichnen gleiche Bezugszeichen gleiche Bauteile bzw. gleiche Merkmale, so dass eine in Bezug auf eine Figur durchgeführte Beschreibung bezüglich eines Bauteils auch für die anderen Figuren gilt, sodass eine wiederholende Beschreibung vermieden wird. Ferner sind einzelne Merkmale, die in Zusammenhang mit einer Ausführungsform beschrieben wurden, auch separat in anderen Ausführungsformen verwendbar.

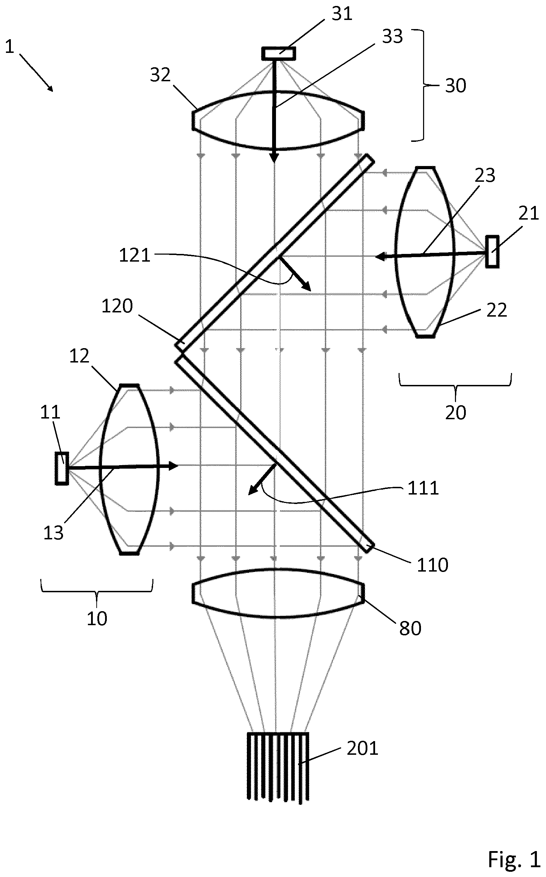

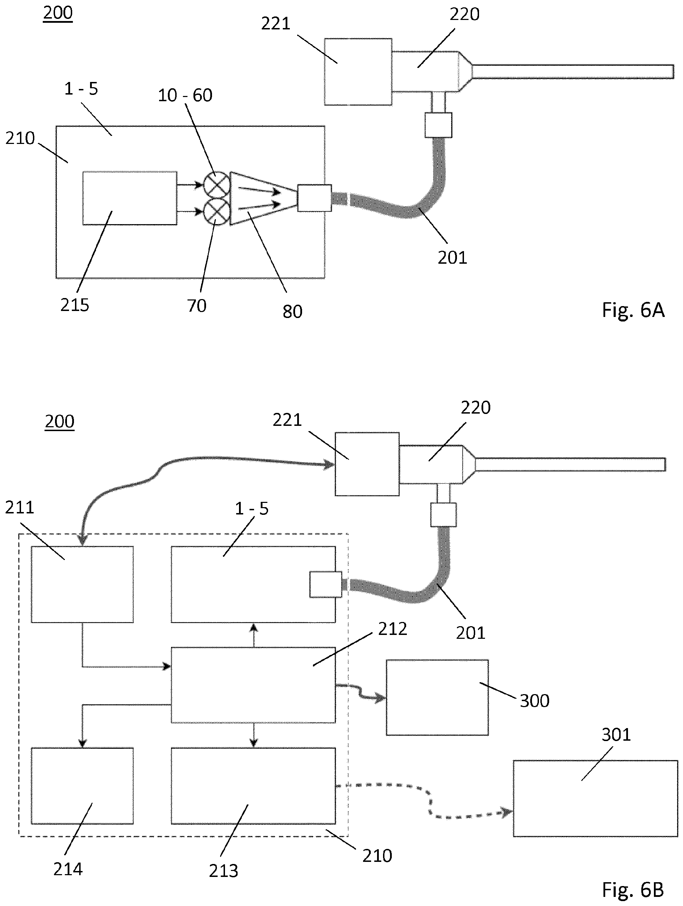

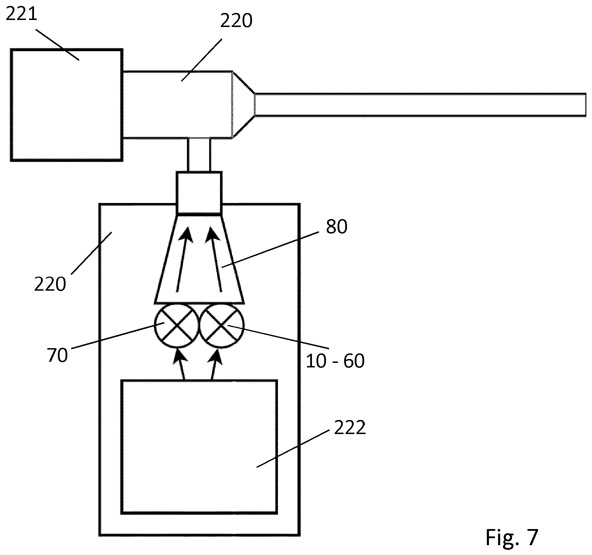

Figur 1 zeigt eine schematische Darstellung einer nicht beanspruchten Beleuchtungsvorrichtung 1. Die Beleuchtungsvorrichtung 1 ist für ein in den Figuren 6A, 6B und 7 dargestelltes Endoskopiesystem 200 vorgesehen. Die Beleuchtungsvorrichtung 1 weist eine erste Leuchteinheit 10 auf, die eine erste Lichtquelle 11 und eine erste Sammeloptik 12 zur Bündelung des von der ersten Lichtquelle 11 emittierten Lichts umfasst, wobei die erste Lichtquelle 11 dazu ausgebildet ist, Licht in einem Zentralwellenlängenbereich zwischen 410 nm und 430 nm und einer Halbwertsbreite von weniger als 20 nm zu emittieren. Die Beleuchtungsvorrichtung 1 weist ferner eine zweite Leuchteinheit 20 auf, die eine zweite Lichtquelle 21 und eine zweite Sammeloptik 22 zur Bündelung des von der zweiten Lichtquelle 21 emittierten Lichts umfasst, wobei die zweite Lichtquelle 21 dazu ausgebildet ist, Licht in einem Zentralwellenlängenbereich zwischen 535 nm und 580 nm und einer Halbwertsbreite von weniger als 120 nm zu emittieren. Weiterhin weist die Beleuchtungsvorrichtung 1 eine dritte Leuchteinheit 30 auf, die eine dritte Lichtquelle 31 und eine dritte Sammeloptik 32 zur Bündelung des von der dritten Lichtquelle 31 emittierten Lichts umfasst, wobei die dritte Lichtquelle 31 dazu ausgebildet ist, Licht in einem Zentralwellenlängenbereich zwischen 620 nm und 650 nm und einer Halbwertsbreite von weniger als 30 nm zu emittieren. Weiterhin weist die Beleuchtungsvorrichtung 1 eine Einkoppeloptik 80 zur Einkopplung des von sämtlichen Leuchteinheiten 10, 20, 30 erzeugten Lichts in ein Endoskopiesystem 200 auf.

Die erste Lichtquelle 11 ist als erste Leuchtdiode 11 und die erste Sammeloptik 12 ist als erste Kollimationsoptik 12 ausgestaltet. Die zweite Lichtquelle 21 kann als zweite Leuchtdiode 22 ausgestaltet sein. Ferner kann die zweite Lichtquelle 21 eine Leuchtdiode 22 und einen Lichtkonverter aufweisen, der die von der zweiten Leuchtdiode 22 emittierte Strahlung in Strahlung im Wellenlängenbereich zwischen 535 nm und 580 nm konvertiert. Die zweite Sammeloptik 22 ist als zweite Kollimationsoptik 22 ausgestaltet. Die dritte Lichtquelle 31 ist als dritte Leuchtdiode 31 und die dritte Sammeloptik 32 ist als dritte Kollimationsoptik 32 ausgestaltet. Jedoch ist die vorliegende Erfindung nicht auf diese entsprechende Ausgestaltung der Lichtquellen 11, 21, 31 und der Sammeloptiken 12, 22, 32 beschränkt.

Bei der in Figur 1 dargestellten Ausführungsform weist die Beleuchtungsvorrichtung 1 zwei dichroitische Spiegel 110, 120 auf. Dabei schließt ein erster dichroitischer Spiegel 110 mit einem zweiten dichroitischen Spiegel 120 einen Winkel von 90° ein.

Folglich stehen auch die Flächennormale 111 des ersten dichroitischen Spiegels 110 und die Flächennormale 121 des zweiten dichroitischen Spiegel 120 senkrecht zueinander.

Die erste Leuchteinheit 10 ist derart zu dem ersten dichroitischen Spiegel 110 orientiert, dass die optische Achse 13 der ersten Leuchteinheit 10 und folglich auch das von der ersten Kollimationsoptik 12 gebündelte Licht unter 45° auf eine Oberfläche des ersten dichroitischen Spiegels 110 treffen. Die optische Achse 13 der ersten Leuchteinheit 10 schließt mit der Flächennormalen 111 des ersten dichroitischen Spiegel 110 einen Winkel von 45° ein. Die zweite Leuchteinheit 20 ist derart zu dem zweiten dichroitischen Spiegel 120 orientiert, dass die optische Achse 23 der zweiten Leuchteinheit 20 und folglich auch das von der zweiten Kollimationsoptik 22 gebündelte Licht unter 45° auf eine Oberfläche des zweiten dichroitischen Spiegels 120 treffen. Die optischen Achsen 13 und 23 der ersten Leuchteinheit 10 und der zweiten Leuchteinheit 20 verlaufen parallel und entgegengesetzt zueinander. Die zweite Leuchteinheit 30 ist derart zu dem zweiten dichroitischen Spiegel 120 orientiert, dass die optische Achse 33 der dritten Leuchteinheit 30 und folglich auch das von der dritten Kollimationsoptik 32 gebündelte Licht unter 45° auf eine Oberfläche des zweiten dichroitischen Spiegels 120 und auf eine Oberfläche des ersten dichroitischen Spiegels 110 treffen. Die optische Achse 33 der dritten Leuchteinheit 30 steht sowohl senkrecht zu der optischen Achse 23 der zweiten Leuchteinheit 20 und zur optischen Achse 13 der ersten Leuchteinheit 10.

Der erste dichroitischer Spiegel 110 ist derart ausgebildet, dass dieser das von der ersten Leuchteinheit 10 emittierte Licht reflektiert und das von der zweiten Leuchteinheit 20 und das von der dritten Leuchteinheit 30 emittierte Licht transmittiert. Der zweite dichroitischer Spiegel 120 ist derart ausgebildet, dass dieser das von der zweiten Leuchteinheit 20 emittierte Licht reflektiert und das von der dritten Leuchteinheit 30 emittierte Licht transmittiert. Folglich sind die Leuchteinheiten 10, 20, 30 und die dichroitischen Spiegel 110, 120 derart zueinander angeordnet, dass das von den Leuchteinheiten 10, 20, 30 emittierte Licht mittels der zwei dichroitischen Spiegel 110, 120 in die Einkoppeloptik 80 eingekoppelt wird.

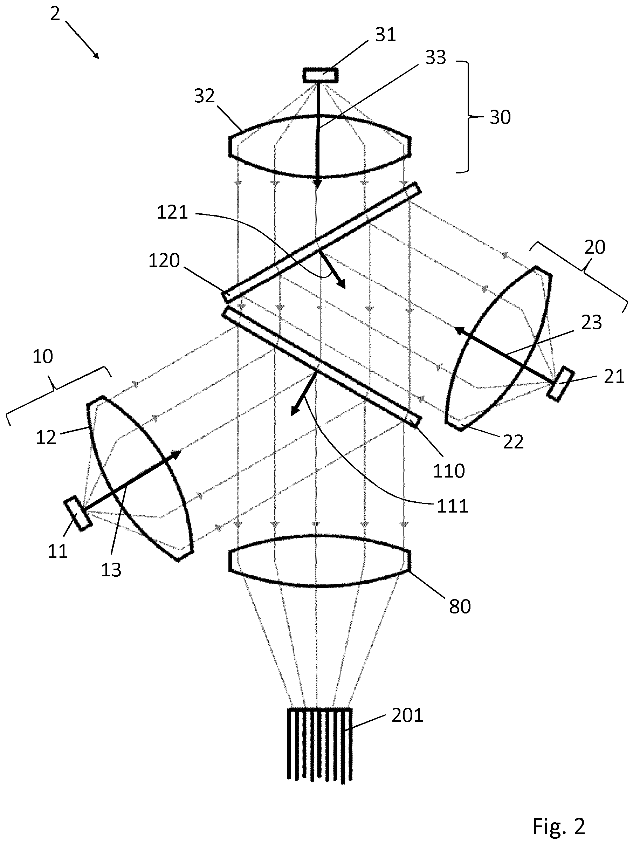

Figur 2 zeigt eine schematische Darstellung einer Beleuchtungsvorrichtung 2 gemäß einer zweiten Ausführungsform der vorliegenden Erfindung. Die in Figur 2 dargestellte Beleuchtungsvorrichtung 2 weist viele Gemeinsamkeiten zu der in Figur 1 dargestellten Beleuchtungsvorrichtung 1 auf. Folglich werden zur Vermeidung von Wiederholungen im Folgenden lediglich die Unterschiede zu der in Figur 1 dargestellten Beleuchtungsvorrichtung 1 beschrieben, da die übrigen Merkmale identisch zu der in Figur 1 dargestellten Beleuchtungsvorrichtung 1 sind.

Bei der in Figur 2 dargestellten Ausführungsform der Beleuchtungsvorrichtung 2 schließen der erste dichroitische Spiegel 110 und der zweite dichroitische Spiegel 120 einen Winkel von weniger als 90°, nämlich einen Winkel von 60° miteinander ein.

Die optische Achse 33 der dritten Leuchteinheit 30 durchdringt den ersten dichroitischen Spiegel 110 und den zweiten dichroitischen Spiegel 120. Die erste Leuchteinheit 10 ist derart zu dem ersten dichroitischen Spiegel 110 orientiert, dass die optische Achse 13 der ersten Leuchteinheit 10 mit der Flächennormalen 111 des ersten dichroitischen Spiegels 110 einen Winkel von 30° einschließt. Die zweite Leuchteinheit 20 ist derart zu dem zweiten dichroitischen Spiegel 120 orientiert, dass die optische Achse 23 der zweiten Leuchteinheit 20 mit der Flächennormalen 121 des zweiten dichroitischen Spiegels 120 einen Winkel von 30° einschließt. Die optischen Achsen 13 und 23 der ersten Leuchteinheit 10 und der zweiten Leuchteinheit 20 schlie-ßen miteinander einen Winkel von 120° ein. Die dritte Leuchteinheit 30 ist bezüglich der ersten Leuchteinheit 10 und der zweiten Leuchteinheit 20 derart angeordnet, dass die optische Achse 33 der der dritten Leuchteinheit 30 sowohl mit der optischen Achse 23 der zweiten Leuchteinheit 20 und mit der optischen Achse 13 der ersten Leuchteinheit 10 jeweils einen Winkel von 120° einschließt.

Die optische Achse 13 der ersten Leuchteinheit 10 und die optische Achse 23 der zweiten Leuchteinheit 20 schließen einen Winkel von 120° miteinander ein. Die optische Achse 13 der ersten Leuchteinheit 10 und die optische Achse 33 der dritten Leuchteinheit 30 schließen einen Winkel von 120° miteinander ein. Die optische Achse 33 der dritten Leuchteinheit 30 und die optische Achse 23 der zweiten Leuchteinheit 20 schließen einen Winkel von 120° miteinander ein.

Die optische Achse 23 der zweiten Leuchteinheit 20 verläuft senkrecht zur Flächennormalen 111 des ersten dichroitischen Spiegels 110, so dass die kollimierten Lichtstrahlen der zweiten Leuchteinheit 20 parallel zur Oberfläche des ersten dichroitischen Spiegels 110 verlaufen. Die optische Achse 13 der ersten Leuchteinheit 13 verläuft senkrecht zur Flächennormalen 121 des zweiten dichroitischen Spiegels 120, so dass die kollimierten Lichtstrahlen der ersten Leuchteinheit 10 parallel zur Oberfläche des zweiten dichroitischen Spiegels 120 verlaufen. Somit ist ein kompakter Aufbau der Beleuchtungsvorrichtung ermöglicht.

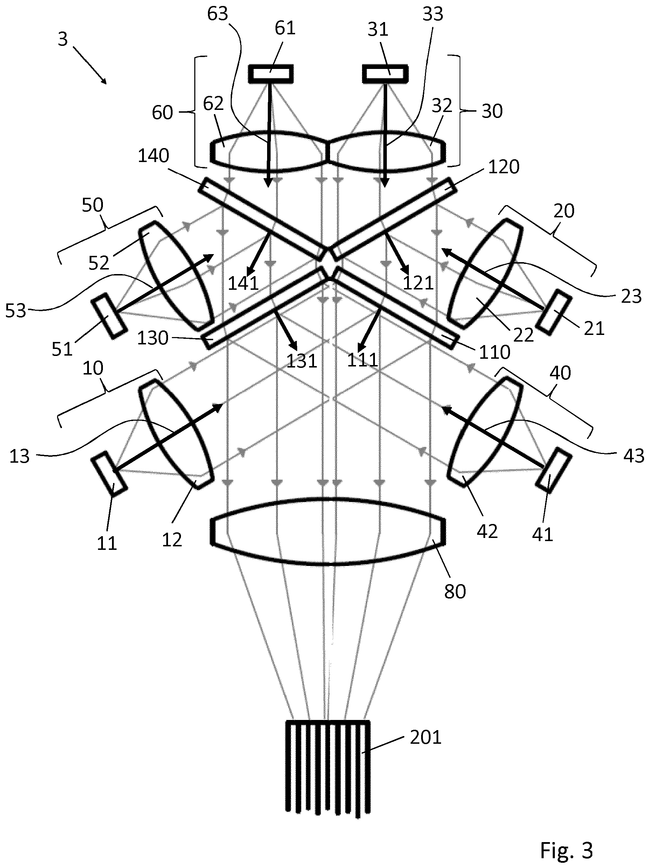

Figur 3 zeigt eine schematische Darstellung einer Beleuchtungsvorrichtung 3 gemäß einer dritten Ausführungsform der vorliegenden Erfindung. Die in Figur 3 dargestellte Beleuchtungsvorrichtung 3 baut auf der in Figur 2 dargestellten Ausführungsform der Beleuchtungsvorrichtung 2 auf, so dass auf die obige Beschreibung zur Beleuchtungsvorrichtung 2 Bezug genommen wird. Im Folgenden werden die noch zur Beleuchtungsvorrichtung 2 zusätzlichen Merkmale der Beleuchtungsvorrichtung 3 beschrieben.

Die Beleuchtungsvorrichtung 3 weist eine vierte Leuchteinheit 40 auf, die eine vierte Lichtquelle 41 und eine vierte Sammeloptik 43 zur Bündelung des von der vierten Lichtquelle 41 emittierten Lichts umfasst, wobei die vierte Lichtquelle 41 dazu ausgebildet ist, Licht in einem Zentralwellenlängenbereich zwischen 470 nm und 490 nm und einer Halbwertsbreite von weniger als 30 nm zu emittieren. Die Beleuchtungsvorrichtung 3 weist ferner eine fünfte Leuchteinheit 50 auf, die eine fünfte Lichtquelle 51 und eine fünfte Sammeloptik 52 zur Bündelung des von der fünften Lichtquelle 51 emittierten Lichts umfasst, wobei die fünfte Lichtquelle 51 dazu ausgebildet ist, Licht in einem Zentralwellenlängenbereich zwischen 580 nm und 620 nm und einer Halbwertsbreite von weniger als 120 nm zu emittieren. Weiterhin weist die Beleuchtungsvorrichtung 3 eine sechste Leuchteinheit 60 auf, die eine sechste Lichtquelle 61 und eine sechste Sammeloptik 62 zur Bündelung des von der sechsten Lichtquelle 61 emittierten Lichts umfasst, wobei die sechste Lichtquelle 61 dazu ausgebildet ist, Licht in einem Zentralwellenlängenbereich zwischen 650 nm und 670 nm und einer Halbwertsbreite von weniger als 30 nm zu emittieren.

Die vierte Lichtquelle 41 ist als vierte Leuchtdiode 41 und die vierte Sammeloptik 42 ist als vierte Kollimationsoptik 42 ausgestaltet. Die fünfte Lichtquelle 51 kann als fünfte Leuchtdiode 52 ausgestaltet sein. Ferner kann die fünfte Lichtquelle 51 eine Leuchtdiode 52 und einen Lichtkonverter aufweisen, der die von der fünften Leuchtdiode 52 emittierte Strahlung in Strahlung im Wellenlängenbereich zwischen 580 nm und 620 nm konvertiert. Die fünfte Sammeloptik 52 ist als fünfte Kollimationsoptik 52 ausgestaltet. Die sechste Lichtquelle 61 ist als sechste Leuchtdiode 61 und die sechste Sammeloptik 62 ist als sechste Kollimationsoptik 62 ausgestaltet. Jedoch ist die vorliegende Erfindung nicht auf diese entsprechende Ausgestaltung der Lichtquellen 41, 51, 61 und der Sammeloptiken 42, 52, 62 beschränkt.

Die Beleuchtungsvorrichtung 3 weist zwei weitere dichroitische Spiegel auf, nämlich einen dritten dichroitischen Spiegel 130 und einen vierten dichroitischen Spiegel 140 auf. Der dritte dichroitische Spiegel 130 schließt mit dem vierten dichroitischen Spiegel 140 einen Winkel von weniger als 90°, nämlich einen Winkel von 60° ein.

Der dritte dichroitische Spiegel 130 und der der vierte dichroitische Spiegel 140 sind die zwischen der Einkoppeloptik 80 und den vierten bis sechsten Leuchteinheiten 40, 50, 60 derart angeordnet sind, dass das von den vierten bis sechsten Leuchteinheiten 40, 50, 60 emittierte Licht mittels der dritten und vierten dichroitischen Spiegel 130, 140 in die Einkoppeloptik 80 eingekoppelt wird.

Die optische Achse 63 der sechsten Leuchteinheit 60 durchdringt den dritten dichroitischen Spiegel 130 und den vierten dichroitischen Spiegel 140. Die vierte Leuchteinheit 40 ist derart zu dem dritten dichroitischen Spiegel 130 orientiert, dass die optische Achse 43 der vierten Leuchteinheit 40 mit der Flächennormalen 131 des dritten dichroitischen Spiegels 130 einen Winkel von 30° einschließt. Die fünfte Leuchteinheit 50 ist derart zu dem vierten dichroitischen Spiegel 140 orientiert, dass die optische Achse 53 der fünften Leuchteinheit 50 mit der Flächennormalen 141 des vierten dichroitischen Spiegels 140 einen Winkel von 30° einschließt. Die optischen Achsen 43 und 53 der vierten Leuchteinheit 40 und der fünften Leuchteinheit 50 schließen miteinander einen Winkel von 120° ein. Die sechste Leuchteinheit 60 ist bezüglich der vierten Leuchteinheit 40 und der fünften Leuchteinheit 50 derart angeordnet, dass die optische Achse 63 der der sechsten Leuchteinheit 60 sowohl mit der optischen Achse 53 der fünften Leuchteinheit 50 und mit der optischen Achse 43 der vierten Leuchteinheit 40 jeweils einen Winkel von 120° einschließt.

Die optische Achse 43 der vierten Leuchteinheit 40 und die optische Achse 53 der fünften Leuchteinheit 50 schließen einen Winkel von 120° miteinander ein. Die optische Achse 43 der vierten Leuchteinheit 40 und die optische Achse 63 der sechsten Leuchteinheit 60 schließen einen Winkel von 120° miteinander ein. Die optische Achse 63 der sechsten Leuchteinheit 60 und die optische Achse 53 der fünften Leuchteinheit 50 schließen einen Winkel von 120° miteinander ein.

Die optische Achse 53 der fünften Leuchteinheit 53 verläuft senkrecht zur Flächennormalen 131 des dritten dichroitischen Spiegels 130, so dass die kollimierten Lichtstrahlen der fünften Leuchteinheit 50 parallel zur Oberfläche des dritten dichroitischen Spiegels 130 verlaufen. Die optische Achse 43 der vierten Leuchteinheit 43 verläuft senkrecht zur Flächennormalen 141 des vierten dichroitischen Spiegels 140, so dass die kollimierten Lichtstrahlen der vierten Leuchteinheit 40 parallel zur Oberfläche des vierten dichroitischen Spiegels 140 verlaufen. Somit ist ein kompakter Aufbau der Beleuchtungsvorrichtung ermöglicht.

Der dritte dichroitischer Spiegel 130 ist derart ausgebildet, dass dieser das von der vierten Leuchteinheit 40 emittierte Licht reflektiert und das von der fünften Leuchteinheit 50 und das von der sechsten Leuchteinheit 60 emittierte Licht transmittiert. Der vierte dichroitischer Spiegel 140 ist derart ausgebildet, dass dieser das von der fünften Leuchteinheit 50 emittierte Licht reflektiert und das von der sechsten Leuchteinheit 60 emittierte Licht transmittiert. Folglich sind die Leuchteinheiten 40, 50, 60 und die dichroitischen Spiegel 130, 140 derart zueinander angeordnet, dass das von den Leuchteinheiten 40, 50, 60 emittierte Licht mittels der zwei dichroitischen Spiegel 130, 140 in die Einkoppeloptik 80 eingekoppelt wird.

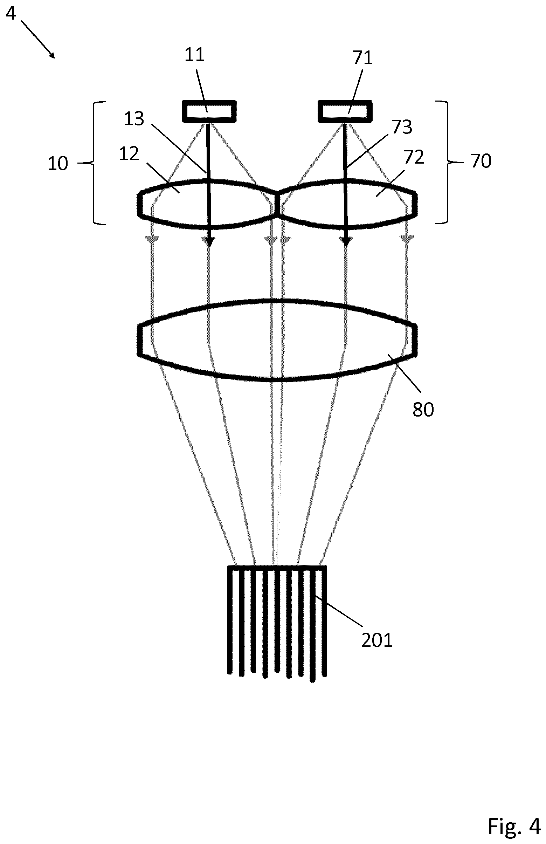

Figur 4 zeigt eine schematische Darstellung einer Beleuchtungsvorrichtung 4 gemäß einer vierten Ausführungsform der vorliegenden Erfindung. Die Beleuchtungsvorrichtung 4 ist für ein in den Figuren 6A, 6B und 7 dargestelltes Endoskopiesystem 200 vorgesehen. Die Beleuchtungsvorrichtung 4 weist eine erste Leuchteinheit 10 auf, die eine erste Lichtquelle 11 und eine erste Sammeloptik 12 zur Bündelung des von der ersten Lichtquelle 11 emittierten Lichts umfasst, wobei die erste Lichtquelle 11 dazu ausgebildet ist, Licht in einem Zentralwellenlängenbereich zwischen 410 nm und 430 nm und einer Halbwertsbreite von weniger als 20 nm zu emittieren. Die Beleuchtungsvorrichtung 4 weist ferner eine Weißlicht-Leuchteinheit 70 auf, die eine Weißlichtquelle 71 und eine Weißlicht-Sammeloptik 72 zur Bündelung des von der Weißlichtquelle 71 emittierten Lichts umfasst, wobei die Weißlichtquelle 71 dazu ausgebildet ist, breitbandiges Weißlicht zu emittieren. Die Beleuchtungsvorrichtung 4 weist ferner eine zweite Leuchteinheit 20 auf, die eine zweite Lichtquelle 21 und eine zweite Sammeloptik 22 zur Bündelung des von der zweiten Lichtquelle 21 emittierten Lichts umfasst, wobei die zweite Lichtquelle 21 dazu ausgebildet ist, Licht in einem Zentralwellenlängenbereich zwischen 535 nm und 580 nm und einer Halbwertsbreite von weniger als 120 nm zu emittieren. Weiterhin weist die Beleuchtungsvorrichtung 4 eine dritte Leuchteinheit 30 auf, die eine dritte Lichtquelle 31 und eine dritte Sammeloptik 32 zur Bündelung des von der dritten Lichtquelle 31 emittierten Lichts umfasst, wobei die dritte Lichtquelle 31 dazu ausgebildet ist, Licht in einem Zentralwellenlängenbereich zwischen 620 nm und 650 nm und einer Halbwertsbreite von weniger als 30 nm zu emittieren.

Die zweite Leuchteinheit 20 und die dritte Leuchteinheit 30 sind in der Darstellung von Figur 4 hinter der ersten Leuchteinheit 10 und der Weißlicht-Leuchteinheit 70 angeordnet und daher in Figur 4 nicht sichtbar.

Weiterhin weist die Beleuchtungsvorrichtung 1 eine Einkoppeloptik 80 zur Einkopplung des von sämtlichen Leuchteinheiten 10, 20, 30 und von der Weißlicht-Leuchteinheit 70 erzeugten Lichts in ein Endoskopiesystem 200 auf.

Die erste Lichtquelle 11 ist als erste Leuchtdiode 11 und die erste Sammeloptik 12 ist als erste Kollimationsoptik 12 ausgestaltet. Die zweite Lichtquelle 21 kann als zweite Leuchtdiode 22 ausgestaltet sein. Ferner kann die zweite Lichtquelle 21 eine Leuchtdiode 22 und einen Lichtkonverter aufweisen, der die von der zweiten Leuchtdiode 22 emittierte Strahlung in Strahlung im Wellenlängenbereich zwischen 535 nm und 580 nm konvertiert. Die zweite Sammeloptik 22 ist als zweite Kollimationsoptik 22 ausgestaltet. Die dritte Lichtquelle 31 ist als dritte Leuchtdiode 31 und die dritte Sammeloptik 32 ist als dritte Kollimationsoptik 32 ausgestaltet. Jedoch ist die vorliegende Erfindung nicht auf diese entsprechende Ausgestaltung der Lichtquellen 11, 21, 31 und der Sammeloptiken 12, 22, 32 beschränkt.

Die Weißlichtquelle 71 ist als Weißlicht-Leuchtdiode 71 ausgebildet. Die Weißlicht-Sammeloptik 72 ist als Weißlicht-Kollimationsoptik 72 ausgebildet. Folglich ist die Weißlichtquelle 71 im Brennpunkt der Weißlicht-Kollimationsoptik 72 angeordnet.

Wie aus Figur 4 ersichtlich ist, wird das Licht, das von der ersten Leuchteinheit 10 und von der zweiten Leuchteinheit 20 und von der dritten Leuchteinheit 30 das von der Weißlicht-Leuchteinheit 70 emittierte Licht unmittelbar in die Einkoppeloptik 80 eingekoppelt.

Folglich sind die jeweiligen optischen Achsen 13, 23, 33, 73 der ersten bis dritten Leuchteinheiten 10, 20, 30 und der Weißlicht-Leuchteinheit 70 parallel versetzt zueinander angeordnet. Weiterhin sind die optischen Achsen 13, 23, 33, 73 der ersten bis dritten Leuchteinheiten 10, 20, 30 und der Weißlicht-Leuchteinheit 70 parallel zur optischen Achse der Einkoppeloptik ausgerichtet.

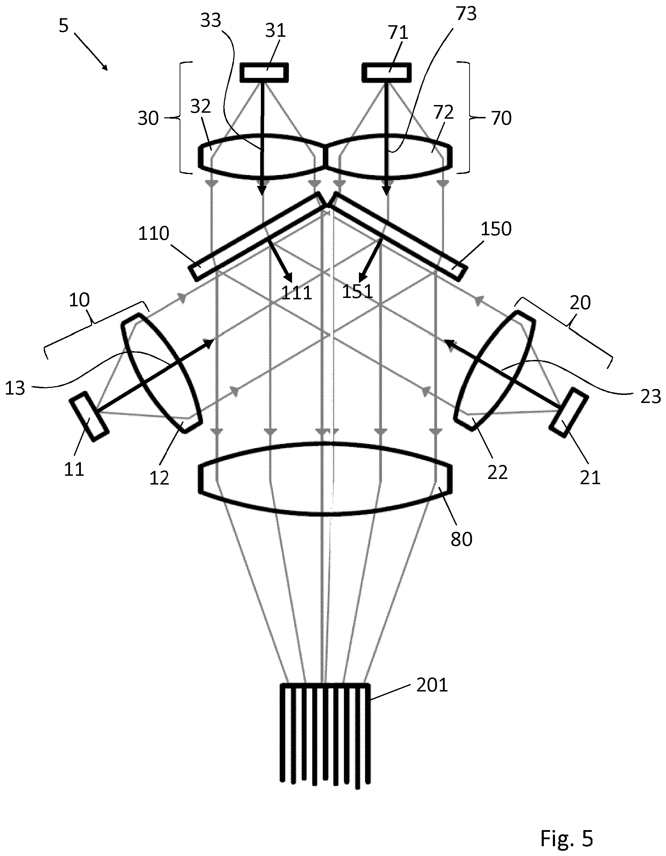

Figur 5 zeigt eine schematische Darstellung einer Beleuchtungsvorrichtung 5 gemäß einer fünften Ausführungsform der vorliegenden Erfindung.

Die Beleuchtungsvorrichtung 5 ist für ein in den Figuren 6A, 6B und 7 dargestelltes Endoskopiesystem 200 vorgesehen. Die Beleuchtungsvorrichtung 5 weist eine erste Leuchteinheit 10 auf, die eine erste Lichtquelle 11 und eine erste Sammeloptik 12 zur Bündelung des von der ersten Lichtquelle 11 emittierten Lichts umfasst, wobei die erste Lichtquelle 11 dazu ausgebildet ist, Licht in einem Zentralwellenlängenbereich zwischen 410 nm und 430 nm und einer Halbwertsbreite von weniger als 20 nm zu emittieren. Die Beleuchtungsvorrichtung 5 weist ferner eine zweite Leuchteinheit 20 auf, die eine zweite Lichtquelle 21 und eine zweite Sammeloptik 22 zur Bündelung des von der zweiten Lichtquelle 21 emittierten Lichts umfasst, wobei die zweite Lichtquelle 21 dazu ausgebildet ist, Licht in einem Zentralwellenlängenbereich zwischen 535 nm und 580 nm und einer Halbwertsbreite von weniger als 120 nm zu emittieren. Weiterhin weist die Beleuchtungsvorrichtung 5 eine dritte Leuchteinheit 30 auf, die eine dritte Lichtquelle 31 und eine dritte Sammeloptik 32 zur Bündelung des von der dritten Lichtquelle 31 emittierten Lichts umfasst, wobei die dritte Lichtquelle 31 dazu ausgebildet ist, Licht in einem Zentralwellenlängenbereich zwischen 620 nm und 650 nm und einer Halbwertsbreite von weniger als 30 nm zu emittieren. Die Beleuchtungsvorrichtung 5 weist ferner eine Weißlicht-Leuchteinheit 70 auf, die eine Weißlichtquelle 71 und eine Weißlicht-Sammeloptik 72 zur Bündelung des von der Weißlichtquelle 71 emittierten Lichts umfasst, wobei die Weißlichtquelle 71 dazu ausgebildet ist, breitbandiges Weißlicht zu emittieren.

Weiterhin weist die Beleuchtungsvorrichtung 5 eine Einkoppeloptik 80 zur Einkopplung des von sämtlichen Leuchteinheiten 10, 20, 30 und von der Weißlicht-Leuchteinheit 70 erzeugten Lichts in ein Endoskopiesystem 200 auf.

Die erste Lichtquelle 11 ist als erste Leuchtdiode 11 und die erste Sammeloptik 12 ist als erste Kollimationsoptik 12 ausgestaltet. Die zweite Lichtquelle 21 kann als zweite Leuchtdiode 22 ausgestaltet sein. Ferner kann die zweite Lichtquelle 21 eine Leuchtdiode 22 und einen Lichtkonverter aufweisen, der die von der zweiten Leuchtdiode 22 emittierte Strahlung in Strahlung im Wellenlängenbereich zwischen 535 nm und 580 nm konvertiert. Die zweite Sammeloptik 22 ist als zweite Kollimationsoptik 22 ausgestaltet. Die dritte Lichtquelle 31 ist als dritte Leuchtdiode 31 und die dritte Sammeloptik 32 ist als dritte Kollimationsoptik 32 ausgestaltet. Jedoch ist die vorliegende Erfindung nicht auf diese entsprechende Ausgestaltung der Lichtquellen 11, 21, 31 und der Sammeloptiken 12, 22, 32 beschränkt.

Die Weißlichtquelle 71 ist als Weißlicht-Leuchtdiode 71 ausgebildet. Die Weißlicht-Sammeloptik 72 ist als Weißlicht-Kollimationsoptik 72 ausgebildet. Folglich ist die Weißlichtquelle 71 im Brennpunkt der Weißlicht-Kollimationsoptik 72 angeordnet.

Die Beleuchtungsvorrichtung 5 weist zwei dichroitische Spiegel 110, 150 aufweist, wobei ein erster dichroitischen Spiegel 110 zwischen der Einkoppeloptik 80 und den Leuchteinheiten 10, 20, 30 und ein fünfter dichroitische Spiegel 150 zwischen der Weißlicht-Leuchteinheit 70 und den Leuchteinheiten 10, 20 und der Einkoppeloptik 80 derart angeordnet sind, dass das von den Leuchteinheiten 10, 20, 30 und von der Weißlicht-Leuchteinheit 70 emittierte Licht mittels der zumindest zwei dichroitischen Spiegel 110, 150 in die Einkoppeloptik 80 eingekoppelt wird.

Der fünfte dichroitischer Spiegel 150 ist derart ausgebildet, dass dieser das von der ersten Leuchteinheit 10 emittierte Licht reflektiert und das von der Weißlicht-Leuchteinheit emittierte Licht transmittiert. Der erste dichroitische Spiegel 110 ist derart ausgebildet, dass dieser das von der zweiten Leuchteinheit 20 emittierte Licht reflektiert und das von der dritten Leuchteinheit 30 emittierte Licht transmittiert.

Aus Figur 5 ist ersichtlich, dass der erste dichroitische Spiegel 110 und der fünfte dichroitische Spiegel 150 einen Winkel von mehr als 90°, nämlich einen Winkel von 120° einschließen.

Ferner ist aus Figur 5 ersichtlich, dass die optische Achse 73 der Weißlicht-Leuchteinheit 70 und die optische Achse 33 der dritten Leuchteinheiten 30 parallel zueinander verlaufen.

Die optische Achse 33 der dritten Leuchteinheit 30 durchdringt den ersten dichroitischen Spiegel 110. Die optische Achse 73 der Weißlicht-Leuchteinheit 70 durchdringt den fünften dichroitischen Spiegel 150 und verläuft parallel versetzt zur optischen Achse 33 der dritten Leuchteinheit 30. Die erste Leuchteinheit 10 ist derart zu dem fünften dichroitischen Spiegel 150 orientiert, dass die optische Achse 13 der ersten Leuchteinheit 10 mit der Flächennormalen 151 des fünften dichroitischen Spiegels 150 einen Winkel von 30° einschließt. Die zweite Leuchteinheit 20 ist derart zu dem fünften dichroitischen Spiegel 150 orientiert, dass die optische Achse 23 der zweiten Leuchteinheit 20 mit der Flächennormalen 151 des fünften dichroitischen Spiegels 150 einen Winkel von 30° einschließt. Die optischen Achsen 13 und 23 der ersten Leuchteinheit 10 und der zweiten Leuchteinheit 20 schließen miteinander einen Winkel von 120° ein. Die dritte Leuchteinheit 30 ist bezüglich der ersten Leuchteinheit 10 und der zweiten Leuchteinheit 20 derart angeordnet, dass die optische Achse 33 der der dritten Leuchteinheit 30 sowohl mit der optischen Achse 23 der zweiten Leuchteinheit 20 und mit der optischen Achse 13 der ersten Leuchteinheit 10 jeweils einen Winkel von 120° einschließt.

Die optische Achse 23 der zweiten Leuchteinheit 23 verläuft senkrecht zur Flächennormalen 151 des fünfen dichroitischen Spiegels 150, so dass die kollimierten Lichtstrahlen der zweiten Leuchteinheit 20 parallel zur Oberfläche des fünften dichroitischen Spiegels 150 verlaufen. Die optische Achse 13 der ersten Leuchteinheit 13 verläuft senkrecht zur Flächennormalen 111 des ersten dichroitischen Spiegels 110, so dass die kollimierten Lichtstrahlen der ersten Leuchteinheit 10 parallel zur Oberfläche des ersten dichroitischen Spiegels 110 verlaufen. Somit ist ein kompakter Aufbau der Beleuchtungsvorrichtung ermöglicht.

Figur 6A zeigt eine schematische Darstellung eines erfindungsgemäßen Endoskopiesystems 200. Das Endoskopiesystem 200 weist eine Beleuchtungsvorrichtung 1 - 5 gemäß einer der oben beschriebenen Ausführungsformen auf. Ferner weist das Endoskopiesystem 200 eine Steuerungseinrichtung auf, die dazu ausgebildet ist, jede der Lichtquellen 11, 21, 31, 41, 51, 61, 71 unabhängig von den jeweils anderen Lichtquellen 11, 21, 31, 41, 51, 61, 71 zu steuern. Die in einer Basisstation 210 integrierte Beleuchtungsvorrichtung 1 - 5 wird mittels einer Stromversorgung 215 mit elektrischer Energie versorgt. Das von der Beleuchtungsvorrichtung 1 - 5 erzeugte Licht wird in einen Lichtleiter 201 mittels der Einkoppeloptik 80 eingekoppelt. Der Lichtleiter 201 mündet in einem Handstück 220, das über ein proximales Ende in einen zu untersuchenden Hohlraum (beispielsweise der Rachen eines Patienten) eingeführt werden kann, und das am distalen Ende eine Kamera und/oder ein Okular aufweist.

Das in Figur 6B dargestellte Endoskopiesystem 200 weist zusätzlich zu den in Figur 6A dargestellten Endoskopiesystem 200 weitere in der Basisstation 210 angeordneten Funktionseinheiten auf. Eine Kameraschnittstelle 211 ermöglicht die Datenkommunikation mit der im Handstück 220 angeordneten Kamera 221. Die Kameraschnittstelle 211 wiederum ist mit einer Videobild-Visualisierungs-Einrichtung 212 datengekoppelt, das wiederum mit einem Sichtgerät 300, beispielsweise einem Bildschirm 300, zur Darstellung des mittels der Kamera 221 gewonnenen Bildern datengekoppelt ist. Die Videobild-Visualisierungs-Einrichtung 212 ist zum Speichern von Bilddaten mit einem Datenspeicher 214 datengekoppelt. Ferner ist die Videobild-Visualisierungs-Einrichtung 212 über eine Netzwerk-Schnittstelle 213 mit einem Praxis-/ Klinik-Informationssystem verbunden.

Figur 7 zeigt ein Endoskopiesystem 200 gemäß einer weiteren Ausführungsform der vorliegenden Erfindung. Bei dem in Figur 7 dargestellten Endoskopiesystem 200 ist die Beleuchtungsvorrichtung 1 - 5 in dem Handstück 220 des Endoskopiesystems 200 integriert. Ferner weist das in Figur 7 dargestellte Endoskopiesystem 200 eine im Handstück 220 integrierte elektrische Energiespeichereinrichtung 222 zur Versorgung der Beleuchtungsvorrichtung 1 - 5 und/oder der Kamera 221 mit elektrischem Strom auf.

Das entsprechend ausgebildete Endoskopiesystem 200 weist unter anderem den Vorteil auf, dass an einem optischen Ausgang des Endoskopiesystems 200 eine erhöhte optische Leistung ankommt, so dass die Bildgebung (sowohl NBI als auch Weißlicht-Bildgebung) verbessert ermöglicht ist. Denn durch die Integration der Beleuchtungsvorrichtung 1 - 5 in dem Handstück 221 des Endoskopiesystems 200 treten verminderte Verluste bei der Einkopplung des von der Beleuchtungsvorrichtung 1 - 5 erzeugten Lichts auf. Beispielsweise kann eine Einkopplung des von der Beleuchtungsvorrichtung 1 - 5 erzeugten Lichts in eine Lichtleitfaser oder in ein Bündel von Lichtleitfasern gänzlich vermieden werden. Ferner ist das entsprechend ausgebildete Endoskopiesystem 200 einfacher und komfortabler handhabbar, da auch auf ein Stromversorgungskabel zwischen dem Handgerät 221 und einer Basisstation des Endoskopiesystems 200 verzichtet werden kann.

### Bezugszeichenliste

- 1 - 5: Beleuchtungsvorrichtung
- 10: erste Leuchteinheit
- 11: erste Lichtquelle
- 12: erste Sammeloptik / Kollimationsoptik
- 13: optische Achse (der ersten Leuchteinheit)
- 20: zweite Leuchteinheit
- 21: zweite Lichtquelle
- 22: zweite Sammeloptik / Kollimationsoptik
- 23: optische Achse (der zweiten Leuchteinheit)
- 30: dritte Leuchteinheit
- 31: dritte Lichtquelle
- 32: dritte Sammeloptik / Kollimationsoptik
- 33: optische Achse (der dritten Leuchteinheit)
- 40: vierte Leuchteinheit
- 41: vierte Lichtquelle
- 42: vierte Sammeloptik / Kollimationsoptik
- 43: optische Achse (der vierten Leuchteinheit)
- 50: fünfte Leuchteinheit
- 51: fünfte Lichtquelle
- 52: fünfte Sammeloptik / Kollimationsoptik
- 43: optische Achse (der fünften Leuchteinheit)
- 60: sechste Leuchteinheit
- 61: sechste Lichtquelle
- 62: sechste Sammeloptik / Kollimationsoptik
- 63: optische Achse (der sechsten Leuchteinheit)
- 70: Weißlicht-Leuchteinheit
- 71: Weißlichtquelle
- 72: Weißlicht-Sammeloptik / Weißlicht-Kollimationsoptik
- 73: optische Achse (der Weißlicht-Leuchteinheit)
- 80: Einkoppeloptik
- 83: optische Achse (der Einkoppeloptik)
- 110: (erste) dichroitischer Spiegel
- 111: Flächennormale (des ersten dichroitischen Spiegels)
- 120: (zweiter) dichroitischer Spiegel
- 121: Flächennormale (des zweiten dichroitischen Spiegels)
- 130: (dritter) dichroitischer Spiegel
- 131: Flächennormale (des dritten dichroitischen Spiegels)
- 140: (vierter) dichroitischer Spiegel
- 141: Flächennormale (des vierten dichroitischen Spiegels)
- 150: (fünfter) dichroitischer Spiegel
- 151: Flächennormale (des fünften dichroitischen Spiegels)
- 200: Endoskopiesystem
- 201: Lichtleiter / Lichtleitfaser / Bündel von Lichtleitfasern
- 210: Basisstation (des Endoskopiesystems)
- 211: Kamera-Schnittstelle
- 212: Videobild-Visualisierungs-Einrichtung
- 213: Netzwerk-Schnittstelle
- 214: Datenspeicher
- 215: Stromversorgung für Leuchteinheiten
- 220: Handstück (des Endoskopiesystems)
- 221: Kamera / Okular (des Handstücks)
- 222: elektrische Energiespeichereinrichtung / Akkumulator
- 300: Sichtgerät (Bildschirm)
- 301: Praxis-/ Klinik-Informationssystem (KIS / PACS)

## Patentansprüche

1. Beleuchtungsvorrichtung (1 - 5) für ein Endoskopiesystem (200), umfassend:
- eine erste Leuchteinheit (10), die eine erste Lichtquelle (11) und eine erste Sammeloptik (12) zur Bündelung des von der ersten Lichtquelle (11) emittierten Lichts umfasst, wobei die erste Lichtquelle (11) dazu ausgebildet ist, Licht in einem Zentralwellenlängenbereich zwischen 410 nm und 430 nm und einer Halbwertsbreite von weniger als 20 nm zu emittieren;
- eine zweite Leuchteinheit (20), die eine zweite Lichtquelle (21) und eine zweite Sammeloptik (22) zur Bündelung des von der zweiten Lichtquelle (21) emittierten Lichts umfasst, wobei die zweite Lichtquelle (21) dazu ausgebildet ist, Licht in einem Zentralwellenlängenbereich zwischen 535 nm und 580 nm und einer Halbwertsbreite von weniger als 120 nm zu emittieren;
- eine dritte Leuchteinheit (30), die eine dritte Lichtquelle (31) und eine dritte Sammeloptik (32) zur Bündelung des von der dritten Lichtquelle (31) emittierten Lichts umfasst, wobei die dritte Lichtquelle (31) dazu ausgebildet ist, Licht in einem Zentralwellenlängenbereich zwischen 620 nm und 650 nm und einer Halbwertsbreite von weniger als 30 nm zu emittieren; und
- eine Einkoppeloptik (80) zur Einkopplung des von sämtlichen Leuchteinheiten (10, 20, 30, 40, 50, 60, 70) erzeugten Lichts in das Endoskopiesystem (200),
wobei
- die Beleuchtungsvorrichtung (1, 2, 3, 5) zumindest zwei dichroitische Spiegel (110, 120, 130, 140, 150) aufweist, die zwischen der Einkoppeloptik (80) und den Leuchteinheiten (10, 20, 30, 40, 50, 60, 70) derart angeordnet sind, dass das von den Leuchteinheiten (10, 20, 30, 40, 50, 60, 70) emittierte Licht mittels der zumindest zwei dichroitischen Spiegel (110, 120, 130, 140, 150) in die Einkoppeloptik (80) eingekoppelt wird, wobei die Beleuchtungsvorrichtung (1 - 5) **dadurch gekennzeichnet ist,**
- **dass** die zumindest zwei dichroitischen Spiegel (110, 120, 130, 140) einen Winkel von 60° einschließen.

2. Beleuchtungsvorrichtung (1 - 5) für ein Endoskopiesystem (200), umfassend:
- eine erste Leuchteinheit (10), die eine erste Lichtquelle (11) und eine erste Sammeloptik (12) zur Bündelung des von der ersten Lichtquelle (11) emittierten Lichts umfasst, wobei die erste Lichtquelle (11) dazu ausgebildet ist, Licht in einem Zentralwellenlängenbereich zwischen 410 nm und 430 nm und einer Halbwertsbreite von weniger als 20 nm zu emittieren;
- eine zweite Leuchteinheit (20), die eine zweite Lichtquelle (21) und eine zweite Sammeloptik (22) zur Bündelung des von der zweiten Lichtquelle (21) emittierten Lichts umfasst, wobei die zweite Lichtquelle (21) dazu ausgebildet ist, Licht in einem Zentralwellenlängenbereich zwischen 535 nm und 580 nm und einer Halbwertsbreite von weniger als 120 nm zu emittieren;
- eine dritte Leuchteinheit (30), die eine dritte Lichtquelle (31) und eine dritte Sammeloptik (32) zur Bündelung des von der dritten Lichtquelle (31) emittierten Lichts umfasst, wobei die dritte Lichtquelle (31) dazu ausgebildet ist, Licht in einem Zentralwellenlängenbereich zwischen 620 nm und 650 nm und einer Halbwertsbreite von weniger als 30 nm zu emittieren; und
- eine Einkoppeloptik (80) zur Einkopplung des von sämtlichen Leuchteinheiten (10, 20, 30, 40, 50, 60, 70) erzeugten Lichts in das Endoskopiesystem (200),
wobei
- die Beleuchtungsvorrichtung (1, 2, 3, 5) zumindest zwei dichroitische Spiegel (110, 120, 130, 140, 150) aufweist, die zwischen der Einkoppeloptik (80) und den Leuchteinheiten (10, 20, 30, 40, 50, 60, 70) derart angeordnet sind, dass das von den Leuchteinheiten (10, 20, 30, 40, 50, 60, 70) emittierte Licht mittels der zumindest zwei dichroitischen Spiegel (110, 120, 130, 140, 150) in die Einkoppeloptik (80) eingekoppelt wird, wobei die Beleuchtungsvorrichtung (1 - 5) **dadurch gekennzeichnet ist,**
- **dass** die zumindest zwei dichroitischen Spiegel (110, 120, 130, 140, 150) einen Winkel von 120° einschließen.

3. Beleuchtungsvorrichtung (1 - 5) nach Anspruch 1 oder 2, **dadurch gekennzeichnet, dass** die zweite Lichtquelle (21) zumindest eine zweite Leuchtdiode und einen Lichtkonverter aufweist.

4. Beleuchtungsvorrichtung (1, 2, 3, 5) nach Anspruch 1, **gekennzeichnet durch** die folgenden Merkmale:
- eine der ersten bis dritten Leuchteinheiten (10, 20, 30) ist bezüglich der zwei dichroitischen Spiegel (110, 120) derart angeordnet, dass die optische Achse (13, 23, 33) dieser Leuchteinheit (10, 20, 30) beide dichroitischen Spiegel (110, 120) durchdringt; und
- jede der übrigen Leuchteinheiten (10, 20, 30) ist bezüglich des dichroitischen Spiegel (110, 120), der dazu ausgebildet ist, das von dieser Leuchteinheit (10, 20, 30) emittierte Licht zu reflektieren, so angeordnet, dass die optische Achse (13, 23, 33) dieser Leuchteinheit (10, 20, 30) mit der Flächennormalen (111, 121) dieses dichroitischen Spiegels (110, 120) einen Winkel von 30° einschließt.

5. Beleuchtungsvorrichtung (3) nach einem der vorhergehenden Ansprüche, **gekennzeichnet durch** folgende Merkmale:
- die Beleuchtungsvorrichtung (3) weist eine vierte Leuchteinheit (40) auf, die eine vierte Lichtquelle (41) und eine vierte Sammeloptik (43) zur Bündelung des von der vierten Lichtquelle (41) emittierten Lichts umfasst, wobei die vierte Lichtquelle (41) dazu ausgebildet ist, Licht in einem Zentralwellenlängenbereich zwischen 470 nm und 490 nm und einer Halbwertsbreite von weniger als 30 nm zu emittieren; und/oder
- die Beleuchtungsvorrichtung (3) weist eine fünfte Leuchteinheit (50) auf, die eine fünfte Lichtquelle (51) und eine fünfte Sammeloptik (52) zur Bündelung des von der fünften Lichtquelle (51) emittierten Lichts umfasst, wobei die fünfte Lichtquelle (51) dazu ausgebildet ist, Licht in einem Zentralwellenlängenbereich zwischen 580 nm und 620 nm und einer Halbwertsbreite von weniger als 120 nm zu emittieren; und/oder
- die Beleuchtungsvorrichtung (3) weist eine sechste Leuchteinheit (60) auf, die eine sechste Lichtquelle (61) und eine sechste Sammeloptik (62) zur Bündelung des von der sechsten Lichtquelle (61) emittierten Lichts umfasst, wobei die sechste Lichtquelle (61) dazu ausgebildet ist, Licht in einem Zentralwellenlängenbereich zwischen 650 nm und 670 nm und einer Halbwertsbreite von weniger als 30 nm zu emittieren.

6. Beleuchtungsvorrichtung (3) nach Anspruch 5, **dadurch gekennzeichnet, dass** die fünfte Lichtquelle zumindest eine Leuchtdiode und einen Lichtkonverter aufweist.

7. Beleuchtungsvorrichtung (3) nach Anspruch 5, **gekennzeichnet durch** die folgenden Merkmale:
- die Beleuchtungsvorrichtung (3) weist die vierte Leuchteinheit (40), die fünfte Leuchteinheit (50) und die sechste Leuchteinheit (60) auf; und
- die Beleuchtungsvorrichtung (3) weist zumindest zwei weitere dichroitische Spiegel (130, 140) auf, die zwischen der Einkoppeloptik (80) und den vierten bis sechsten Leuchteinheiten (40, 50, 60) derart angeordnet sind, dass das von den vierten bis sechsten Leuchteinheiten (40, 50, 60) emittierte Licht mittels der zumindest zwei weiteren dichroitischen Spiegel (130, 140) in die Einkoppeloptik (80) eingekoppelt wird.

8. Beleuchtungsvorrichtung (3) nach Anspruch 7, **dadurch gekennzeichnet, dass** die zumindest zwei weiteren dichroitischen Spiegel (130, 140) einen Winkel von weniger als 90° einschließen.

9. Beleuchtungsvorrichtung (3) nach Anspruch 7 oder 8, **dadurch gekennzeichnet, dass** die zumindest zwei weiteren dichroitischen Spiegel (130, 140) einen Winkel von 60° einschließen.

10. Beleuchtungsvorrichtung (3) nach Anspruch 9, **gekennzeichnet durch** die folgenden Merkmale:
- eine der vierten bis sechsten Leuchteinheiten (40, 50, 60) ist bezüglich der zwei weiteren dichroitischen Spiegel (130, 140) derart angeordnet, dass die optische Achse (43, 53, 63) dieser Leuchteinheit (40, 50, 60) beide weiteren dichroitischen Spiegel (130, 140) durchdringt; und
- jede der übrigen vierten bis sechsten Leuchteinheiten (40, 50, 60) ist bezüglich des weiteren dichroitischen Spiegels (130, 140), der dazu ausgebildet ist, das von dieser Leuchteinheit (40, 50, 60) emittierte Licht zu reflektieren, so angeordnet, dass die optische Achse (43, 53, 63) dieser Leuchteinheit (40, 50, 60) mit der Flächennormalen (131, 141) dieses weiteren dichroitischen Spiegels (130, 140) einen Winkel von 30° einschließt.

11. Beleuchtungsvorrichtung (4, 5) nach Anspruch 1 oder 2, **dadurch gekennzeichnet, dass** die Beleuchtungsvorrichtung (4, 5) eine Weißlicht-Leuchteinheit (70) aufweist, die eine Weißlichtquelle (71) und eine Weißlicht-Sammeloptik (72) zur Bündelung des von der Weißlichtquelle (71) emittierten Lichts umfasst, wobei die Weißlichtquelle (71) dazu ausgebildet ist, breitbandiges Weißlicht zu emittieren.

12. Beleuchtungsvorrichtung (4) nach Anspruch 11, **dadurch gekennzeichnet, dass** das von der Weißlicht-Leuchteinheit (70) emittierte Licht unmittelbar in die Einkoppeloptik (80) eingekoppelt wird.

13. Beleuchtungsvorrichtung (5) nach Anspruch 11, **dadurch gekennzeichnet, dass** die Beleuchtungsvorrichtung (5) zumindest zwei dichroitische Spiegel (110, 150) aufweist, wobei einer der dichroitischen Spiegel (110) zwischen der Einkoppeloptik (80) und den Leuchteinheiten (10, 20, 30) und der andere dichroitische Spiegel (150) zwischen der Weißlicht-Leuchteinheit (70) und den Leuchteinheiten (10, 20) und der Einkoppeloptik (80) derart angeordnet sind, dass das von den Leuchteinheiten (10, 20, 30) und von der Weißlicht-Leuchteinheit (70) emittierte Licht mittels der zumindest zwei dichroitischen Spiegel (110, 150) in die Einkoppeloptik (80) eingekoppelt wird.

14. Beleuchtungsvorrichtung (5) nach Anspruch 13, **dadurch gekennzeichnet, dass** eine optische Achse (73) der Weißlicht-Leuchteinheit (70) und eine optische Achse (13, 23, 33) einer der ersten bis dritten Leuchteinheiten (10, 20, 30) parallel zueinander verlaufen.

15. Endoskopiesystem (200), umfassend
- eine Beleuchtungsvorrichtung (1 - 5) nach einem der vorhergehenden Ansprüche; und
- eine Steuerungseinrichtung, die dazu ausgebildet ist, jede der Lichtquellen (11, 21, 31, 41, 51, 61, 71) unabhängig von den jeweils anderen Lichtquellen (11, 21, 31, 41, 51, 61, 71) zu steuern.

16. Endoskopiesystem (200) nach Anspruch 15, **dadurch gekennzeichnet, dass** die Beleuchtungsvorrichtung (1 - 5) in einem Handstück (220) des Endoskopiesystems (200) integriert ist.

17. Endoskopiesystem (200) nach Anspruch 16, **dadurch gekennzeichnet, dass** eine Kamera (221) im Handstück (220) dazu ausgebildet ist, Bilddaten kabellos zu übertragen.

18. Endoskopiesystem (200) nach Anspruch 16 oder 17, **dadurch gekennzeichnet, dass** in dem Handstück (220) eine elektrische Energiespeichereinrichtung (222) zur Versorgung der Beleuchtungsvorrichtung (1 - 5) und/oder der Kamera (221) mit elektrischem Strom integriert ist.

## Claims

1. Illumination device (1 - 5) for an endoscopy system (200), comprising:
- a first lighting unit (10) having a first light source (11) and first collecting optics (12) for focusing the light emitted by the first light source (11), wherein the first light source (11) is designed to emit light in a central wavelength range between 410 nm and 430 nm and a half-width of less than 20 nm;
- a second lighting unit (20) having a second light source (21) and second collecting optics (22) for focusing the light emitted by the second light source (21), wherein the second light source (21) is designed to emit light in a central wavelength range between 535 nm and 580 nm and a half-width of less than 120 nm;
- a third lighting unit (30) having a third light source (31) and third collecting optics (32) for focusing the light emitted by the third light source (31), wherein the third light source (31) is designed to emit light in a central wavelength range between 620 nm and 650 nm and a half-width of less than 30 nm; and
- coupling optics (80) for coupling the light generated by all the lighting units (10, 20, 30, 40, 50, 60, 70) into the endoscopy system (200),
wherein
- the illumination device (1, 2, 3, 5) comprises at least two dichroic mirrors (110, 120, 130, 140, 150) which are arranged between the coupling optics (80) and the lighting units (10, 20, 30, 40, 50, 60, 70) such that the light emitted by the lighting units (10, 20, 30, 40, 50, 60, 70) is coupled into the coupling optics (80) by means of the at least two dichroic mirrors (110, 120, 130, 140, 150), wherein the illumination device (1 - 5) is **characterized in that**
- the at least two dichroic mirrors (110, 120, 130, 140) enclose an angle of 60°.

2. Illumination device (1 - 5) for an endoscopy system (200), comprising:
- a first lighting unit (10) having a first light source (11) and first collecting optics (12) for focusing the light emitted by the first light source (11), wherein the first light source (11) is designed to emit light in a central wavelength range between 410 nm and 430 nm and a half-width of less than 20 nm;
- a second lighting unit (20) having a second light source (21) and second collecting optics (22) for focusing the light emitted by the second light source (21), wherein the second light source (21) is designed to emit light in a central wavelength range between 535 nm and 580 nm and a half-width of less than 120 nm;
- a third lighting unit (30) having a third light source (31) and third collecting optics (32) for focusing the light emitted by the third light source (31), wherein the third light source (31) is designed to emit light in a central wavelength range between 620 nm and 650 nm and a half-width of less than 30 nm; and
- coupling optics (80) for coupling the light generated by all the lighting units (10, 20, 30, 40, 50, 60, 70) into the endoscopy system (200),
wherein
- the illumination device (1, 2, 3, 5) comprises at least two dichroic mirrors (110, 120, 130, 140, 150) which are arranged between the coupling optics (80) and the lighting units (10, 20, 30, 40, 50, 60, 70) such that the light emitted by the lighting units (10, 20, 30, 40, 50, 60, 70) is coupled into the coupling optics (80) by means of the at least two dichroic mirrors (110, 120, 130, 140, 150), wherein the illumination device (1 - 5) is **characterized in that**
- the at least two dichroic mirrors (110, 120, 130, 140, 150) enclose an angle of 120°.

3. Illumination device (1 - 5) according to claim 1 or 2, **characterized in that** the second light source (21) has at least a second LED and a light converter.

4. Illumination device (1, 2, 3, 5) according to claim 1, **characterized by** the following features:
- one of the first to third lighting units (10, 20, 30) is arranged with respect to the two dichroic mirrors (110, 120) such that the optical axis (13, 23, 33) of this lighting unit (10, 20, 30) penetrates both dichroic mirrors (110, 120); and
- each of the remaining lighting units (10, 20, 30) is arranged with respect to the dichroic mirror (110, 120) which is designed to reflect the light emitted by this lighting unit (10, 20, 30) such that the optical axis (13, 23, 33) of this lighting unit (10, 20, 30) encloses an angle of 30° with the surface normal (111, 121) of this dichroic mirror (110, 120).

5. Illumination device (3) according to any of the preceding claims, **characterized by** the following features:
- the illumination device (3) comprises a fourth lighting unit (40) which has a fourth light source (41) and fourth collecting optics (43) for focusing the light emitted by the fourth light source (41), wherein the fourth light source (41) is designed to emit light in a central wavelength range between 470 nm and 490 nm and a half-width of less than 30 nm; and/or
- the illumination device (3) comprises a fifth lighting unit (50) which has a fifth light source (51) and fifth collecting optics (52) for focusing the light emitted by the fifth light source (51), wherein the fifth light source (51) is designed to emit light in a central wavelength range between 580 nm and 620 nm and a half-width of less than 120 nm; and/or
- the illumination device (3) comprises a sixth lighting unit (60) having a sixth light source (61) and sixth collecting optics (62) for focusing the light emitted by the sixth light source (61), wherein the sixth light source (61) is designed to emit light in a central wavelength range between 650 nm and 670 nm and a half-width of less than 30 nm.

6. Illumination device (3) according to claim 5, **characterized in that** the fifth light source has at least one LED and a light converter.

7. Illumination device (3) according to claim 5, **characterized by** the following features:
- the illumination device (3) comprises the fourth lighting unit (40), the fifth lighting unit (50) and the sixth lighting unit (60); and
- the illumination device (3) comprises at least two further dichroic mirrors (130, 140) which are arranged between the coupling optics (80) and the fourth to sixth lighting units (40, 50, 60) such that the light emitted by the fourth to sixth lighting units (40, 50, 60) is coupled into the coupling optics (80) by means of the at least two further dichroic mirrors (130, 140).

8. Illumination device (3) according to claim 7, **characterized in that** the at least two further dichroic mirrors (130, 140) enclose an angle of less than 90°.

9. Illumination device (3) according to claim 7 or 8, **characterized in that** the at least two further dichroic mirrors (130, 140) enclose an angle of 60°.

10. Illumination device (3) according to claim 9, **characterized by** the following features:
- one of the fourth to sixth lighting units (40, 50, 60) is arranged with respect to the two further dichroic mirrors (130, 140) such that the optical axis (43, 53, 63) of this lighting unit (40, 50, 60) penetrates both further dichroic mirrors (130, 140); and
- each of the remaining fourth to sixth lighting units (40, 50, 60) is arranged with respect to the further dichroic mirror (130, 140) which is designed to reflect the light emitted by this lighting unit (40, 50, 60) such that the optical axis (43, 53, 63) of this lighting unit (40, 50, 60) encloses an angle of 30 with the surface normal (131, 141) of this further dichroic mirror (130, 140).

11. Illumination device (4, 5) according to claim 1 or 2, **characterized in that** the illumination device (4, 5) comprises a white-light lighting unit (70) which has a white light source (71) and white-light collecting optics (72) for focusing the light emitted by the white light source (71), wherein the white light source (71) is designed to emit broadband white light.

12. Illumination device (4) according to claim 11, **characterized in that** the light emitted by the white-light lighting unit (70) is directly coupled into the coupling optics (80).

13. Illumination device (5) according to claim 11, **characterized in that** the illumination device (5) comprises at least two dichroic mirrors (110, 150), wherein one of the dichroic mirrors (110) is arranged between the coupling optics (80) and the lighting units (10, 20, 30) and the other dichroic mirror (150) is arranged between the white-light lighting unit (70) and the lighting units (10, 20) and the coupling optics (80) such that the light emitted by the lighting units (10, 20, 30) and by the white-light lighting unit (70) is coupled into the coupling optics (80) by means of the at least two dichroic mirrors (110, 150).

14. Illumination device (5) according to claim 13, **characterized in that** an optical axis (73) of the white-light lighting unit (70) and an optical axis (13, 23, 33) of one of the first to third lighting units (10, 20, 30) extend in parallel with one another.

15. Endoscopy system (200), comprising
- an illumination device (1 - 5) according to any of the preceding claims; and
- a control means designed to control each of the light sources (11, 21, 31, 41, 51, 61, 71) independently of the other light sources (11, 21, 31, 41, 51, 61, 71).

16. Endoscopy system (200) according to claim 15, **characterized in that** the illumination device (1 - 5) is integrated into a handpiece (220) of the endoscopy system (200).

17. Endoscopy system (200) according to claim 16, **characterized in that** a camera (221) in the handpiece (220) is designed to transmit image data wirelessly.

18. Endoscopy system (200) according to claim 16 or 17,
**characterized in that** an electrical energy storage means (222) for supplying electrical current to the illumination device (1 - 5) and/or the camera (221) is integrated into the handpiece (220).

## Revendications

1. Dispositif d'éclairage (1 - 5) pour un système d'endoscopie (200), comprenant :
- une première unité d'éclairage (10) comprenant une première source de lumière (11) et une première optique de convergence (12) destinée à focaliser la lumière émise par la première source de lumière (11), dans lequel la première source de lumière (11) est configurée pour émettre de la lumière dans une gamme de longueurs d'onde centrales comprise entre 410 nm et 430 nm et avec une largeur à mi-hauteur inférieure à 20 nm ;
- une deuxième unité d'éclairage (20) comprenant une deuxième source de lumière (21) et une deuxième optique de convergence (22) destinée à focaliser la lumière émise par la deuxième source de lumière (21), dans lequel la deuxième source de lumière (21) est configurée pour émettre de la lumière dans une gamme de longueurs d'onde centrales comprise entre 535 nm et 580 nm et avec une largeur à mi-hauteur inférieure à 120 nm ;
- une troisième unité d'éclairage (30) comprenant une troisième source de lumière (31) et une troisième optique de convergence (32) destinée à focaliser la lumière émise par la troisième source de lumière (31), dans lequel la troisième source de lumière (31) est configurée pour émettre de la lumière dans une gamme de longueurs d'onde centrales comprise entre 620 nm et 650 nm et avec une largeur à mi-hauteur inférieure à 30 nm ; et
- une optique de couplage (80) destinée à coupler la lumière générée par l'ensemble des unités d'éclairage (10, 20, 30, 40, 50, 60, 70) dans le système d'endoscopie (200),
dans lequel
- le dispositif d'éclairage (1, 2, 3, 5) présente au moins deux miroirs dichroïques (110, 120, 130, 140, 150) qui sont disposés entre l'optique de couplage (80) et les unités d'éclairage (10, 20, 30, 40, 50, 60, 70) de telle sorte que la lumière émise par les unités d'éclairage (10, 20, 30, 40, 50, 60, 70) est couplée dans l'optique de couplage (80) au moyen des au moins deux miroirs dichroïques (110, 120, 130, 140, 150), dans lequel le dispositif d'éclairage (1 - 5) est **caractérisé en ce**
- **que** les au moins deux miroirs dichroïques (110, 120, 130, 140) forment un angle de 60°.

2. Dispositif d'éclairage (1 - 5) pour un système d'endoscopie (200), comprenant :
- une première unité d'éclairage (10) comprenant une première source de lumière (11) et une première optique de convergence (12) destinée à focaliser la lumière émise par la première source de lumière (11), dans lequel la première source de lumière (11) est configurée pour émettre de la lumière dans une gamme de longueurs d'onde centrales comprise entre 410 nm et 430 nm et avec une largeur à mi-hauteur inférieure à 20 nm ;
- une deuxième unité d'éclairage (20) comprenant une deuxième source de lumière (21) et une deuxième optique de convergence (22) destinée à focaliser la lumière émise par la deuxième source de lumière (21), dans lequel la deuxième source de lumière (21) est configurée pour émettre de la lumière dans une gamme de longueurs d'onde centrales comprise entre 535 nm et 580 nm et avec une largeur à mi-hauteur inférieure à 120 nm ;
- une troisième unité d'éclairage (30) comprenant une troisième source de lumière (31) et une troisième optique de convergence (32) destinée à focaliser la lumière émise par la troisième source de lumière (31), dans lequel la troisième source de lumière (31) est configurée pour émettre de la lumière dans une gamme de longueurs d'onde centrales comprise entre 620 nm et 650 nm et avec une largeur à mi-hauteur inférieure à 30 nm ; et
- une optique de couplage (80) destinée à coupler la lumière générée par l'ensemble des unités d'éclairage (10, 20, 30, 40, 50, 60, 70) dans le système d'endoscopie (200),
dans lequel
- le dispositif d'éclairage (1, 2, 3, 5) présente au moins deux miroirs dichroïques (110, 120, 130, 140, 150) qui sont disposés entre l'optique de couplage (80) et les unités d'éclairage (10, 20, 30, 40, 50, 60, 70) de telle sorte que la lumière émise par les unités d'éclairage (10, 20, 30, 40, 50, 60, 70) est couplée dans l'optique de couplage (80) au moyen des au moins deux miroirs dichroïques (110, 120, 130, 140, 150), dans lequel le dispositif d'éclairage (1 - 5) est **caractérisé en ce**
- **que** les au moins deux miroirs dichroïques (110, 120, 130, 140, 150) forment un angle de 120°.

3. Dispositif d'éclairage (1 - 5) selon la revendication 1 ou 2,
**caractérisé en ce que** la deuxième source de lumière (21) présente au moins une seconde diode électroluminescente et un convertisseur de lumière.

4. Dispositif d'éclairage (1, 2, 3, 5) selon la revendication 1,
**caractérisé par** les caractéristiques suivantes :
- une unité d'éclairage parmi les première à troisième unités d'éclairage (10, 20, 30) est disposée par rapport aux deux miroirs dichroïques (110, 120) de telle sorte que l'axe optique (13, 23, 33) de ladite unité d'éclairage (10, 20, 30) traverse les deux miroirs dichroïques (110, 120) ; et
- chacune parmi les unités d'éclairage (10, 20, 30) restantes est disposée par rapport au miroir dichroïque (110, 120) configuré pour réfléchir la lumière émise par ladite unité d'éclairage (10, 20, 30) de sorte que l'axe optique (13, 23, 33) de ladite unité d'éclairage (10, 20, 30) forme un angle de 30° avec la normale à la surface (111, 121) dudit miroir dichroïque (110, 120).

5. Dispositif d'éclairage (3) selon l'une des revendications précédentes, **caractérisé par** les caractéristiques suivantes :
- le dispositif d'éclairage (3) présente une quatrième unité d'éclairage (40) comprenant une quatrième source de lumière (41) et une quatrième optique de convergence (43) destinée à focaliser la lumière émise par la quatrième source de lumière (41), dans lequel la quatrième source de lumière (41) est configurée pour émettre de la lumière dans une gamme de longueurs d'onde centrales comprise entre 470 nm et 490 nm et avec une largeur à mi-hauteur inférieure à 30 nm ; et/ou
- le dispositif d'éclairage (3) présente une cinquième unité d'éclairage (50) comprenant une cinquième source de lumière (51) et une cinquième optique de convergence (52) destinée à focaliser la lumière émise par la cinquième source de lumière (51), dans lequel la cinquième source de lumière (51) est configurée pour émettre de la lumière dans une gamme de longueurs d'onde centrales comprise entre 580 nm et 620 nm et avec une largeur à mi-hauteur inférieure à 120 nm ; et/ou
- le dispositif d'éclairage (3) présente une sixième unité d'éclairage (60) comprenant une sixième source de lumière (61) et une sixième optique de convergence (62) destinée à focaliser la lumière émise par la sixième source de lumière (61), dans lequel la sixième source de lumière (61) est configurée pour émettre de la lumière dans une gamme de longueurs d'onde centrales comprise entre 650 nm et 670 nm et avec une largeur à mi-hauteur inférieure à 30 nm.

6. Dispositif d'éclairage (3) selon la revendication 5, **caractérisé en ce que** la cinquième source de lumière présente au moins une diode électroluminescente et un convertisseur de lumière.

7. Dispositif d'éclairage (3) selon la revendication 5, **caractérisé par** les caractéristiques suivantes :
- le dispositif d'éclairage (3) présente la quatrième unité d'éclairage (40), la cinquième unité d'éclairage (50) et la sixième unité d'éclairage (60) ; et
- le dispositif d'éclairage (3) présente au moins deux autres miroirs dichroïques (130, 140) qui sont disposés entre l'optique de couplage (80) et les quatrième à sixième unités d'éclairage (40, 50, 60) de telle sorte que la lumière émise par les quatrième à sixième unités d'éclairage (40, 50, 60) est couplée dans l'optique de couplage (80) au moyen des au moins deux autres miroirs dichroïques (130, 140).

8. Dispositif d'éclairage (3) selon la revendication 7, **caractérisé en ce que** les au moins deux autres miroirs dichroïques (130, 140) forment un angle inférieur à 90°.

9. Dispositif d'éclairage (3) selon la revendication 7 ou 8,
**caractérisé en ce que** les au moins deux autres miroirs dichroïques (130, 140) forment un angle de 60°.

10. Dispositif d'éclairage (3) selon la revendication 9, **caractérisé par** les caractéristiques suivantes :
- une unité d'éclairage parmi les quatrième à sixième unités d'éclairage (40, 50, 60) est disposée par rapport aux deux autres miroirs dichroïques (130, 140) de telle sorte que l'axe optique (43, 53, 63) de ladite unité d'éclairage (40, 50, 60) traverse les deux autres miroirs dichroïques (130, 140) ; et
- chacune parmi les quatrième à sixième unités d'éclairage (40, 50, 60) restantes est disposée par rapport à l'autre miroir dichroïque (130, 140) configuré pour réfléchir la lumière émise par ladite unité d'éclairage (40, 50, 60) de sorte que l'axe optique (43, 53, 63) de ladite unité d'éclairage (40, 50, 60) forme un angle de 30° avec la normale à la surface (131, 141) dudit autre miroir dichroïque (130, 140).

11. Dispositif d'éclairage (4, 5) selon la revendication 1 ou 2,
**caractérisé en ce que** le dispositif d'éclairage (4, 5) présente une unité d'éclairage à lumière blanche (70) qui comprend une source de lumière blanche (71) et une optique de convergence de lumière blanche (72) destinée à focaliser la lumière émise par la source de lumière blanche (71), dans lequel la source de lumière blanche (71) est configurée pour émettre une lumière blanche à large bande.

12. Dispositif d'éclairage (4) selon la revendication 11,
**caractérisé en ce que** la lumière émise par l'unité d'éclairage à lumière blanche (70) est directement couplée dans l'optique de couplage (80).

13. Dispositif d'éclairage (5) selon la revendication 11,
**caractérisé en ce que** le dispositif d'éclairage (5) présente au moins deux miroirs dichroïques (110, 150), dans lequel l'un des miroirs dichroïques (110) est disposé entre l'optique de couplage (80) et les unités d'éclairage (10, 20, 30) et l'autre miroir dichroïque (150) est disposé entre l'unité d'éclairage à lumière blanche (70) et les unités d'éclairage (10, 20) et l'optique de couplage (80), de telle sorte que la lumière émise par les unités d'éclairage (10, 20, 30) et par l'unité d'éclairage à lumière blanche (70) est couplée dans l'optique de couplage (80) au moyen des au moins deux miroirs dichroïques (110, 150).

14. Dispositif d'éclairage (5) selon la revendication 13,
**caractérisé en ce qu'**un axe optique (73) de l'unité d'éclairage à lumière blanche (70) et un axe optique (13, 23, 33) de l'une des première à troisième unités d'éclairage (10, 20, 30) s'étendent parallèlement l'un à l'autre.

15. Système d'endoscopie (200), comprenant
- un dispositif d'éclairage (1 - 5) selon l'une des revendications précédentes ; et
- un appareil de commande qui est configuré pour commander chacune des sources de lumière (11, 21, 31, 41, 51, 61, 71) indépendamment des autres sources de lumière (11, 21, 31, 41, 51, 61, 71) respectives.

16. Système d'endoscopie (200) selon la revendication 15,
**caractérisé en ce que** le dispositif d'éclairage (1 à 5) est intégré dans une pièce à main (220) du système d'endoscopie (200).

17. Système d'endoscopie (200) selon la revendication 16,
**caractérisé en ce qu'**une caméra (221) dans la pièce à main (220) est configurée pour transmettre des données d'images sans fil.

18. Système d'endoscopie (200) selon la revendication 16 ou 17,
**caractérisé en ce qu'**un appareil de stockage d'énergie électrique (222) destiné à alimenter en courant électrique le dispositif d'éclairage (1 - 5) et/ou la caméra (221) est intégré dans la pièce à main (220).
